# FASCICULE DE BREVET EUROPEEN

(11) **EP 1 735 297 B1**
(45) Date de publication et mention de la délivrance du brevet: **03.10.2007**
(21) Numéro de dépôt: 05753693.0
(22) Date de dépôt: 08.04.2005
(51) Int. Cl.: C07D 327/02, C07C 323/22

(54) **PROCEDE DE SYNTHESE ET INTERMEDIAIRES DE BENZOXATHIEPINES**
SYNTHESEVERFAHREN UND BENZOXATHIEPINZWISCHENPRODUKTE
SYNTHESISING METHOD AND BENZOXATHIEPINE INTERMEDIATES

(30) Priorité: 09.04.2004 FR 0403760
(43) Date de publication de la demande: 27.12.2006
(73) Titulaire: PIERRE FABRE MEDICAMENT, 92100 Boulogne-Billancourt (FR)
(72) Inventeur: VACHER, Bernard, F-81100 Castres (FR); BRUNEL, Yves, F-81150 Marssac-Sur-Tarn (FR); CASTAN-CUISIAT, Florence, F-81100 Castres (FR)
(74) Mandataire: Ahner, Francis
(86) Numéro de dépôt international: PCT/FR2005/000859
(87) Numéro de publication internationale: WO 2005/103027

(56) Documents cités:
- WO-A-02/081464
- US-A- 3 636 074

## Description

La présente invention concerne un nouveau procédé de préparation de dérivés de benzoxathiépines de formule générale 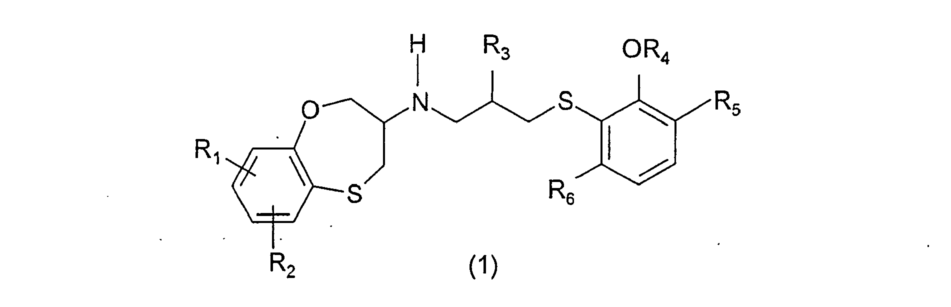 dans laquelle :
R₁ et R₂, identiques ou différents, représentent :
   - un atome d'hydrogène ;
   - un atome de fluor ou un atome de chlore ;
   - un groupe hydroxy ;
   - un radical alkyle choisi parmi les radicaux méthyle, éthyle, propyle ou isopropyle ;
   - un radical alcoxy choisi parmi les radicaux méthoxy, éthyloxy, propyloxy ou isopropyloxy ;
   - lorsque les groupes R₁ et R₂ occupent des positions adjacentes sur le cycle aromatique, alors R₁R₂ représentent -CH₂CH₂CH₂-, -OCH₂CH₂-, -OCH₂O- ou -CH₂CH₂O- ;
R₃ représente :
   - un radical alkyle, choisi parmi les radicaux méthyle, éthyle, propyle ou isopropyle ;
   - un groupe hydroxy ou un radical méthoxy ;
R₄ représente :
   - un atome d'hydrogène ou un radical méthyle ;
R₅ et R₆ identiques ou différents représentent :
   - un atome d'hydrogène ;
   - un radical alkyle choisi parmi les radicaux méthyle, éthyle ou isopropyle ;
   - un radical alcoxy choisi parmi les radicaux méthoxy, éthyloxy ou isopropyloxy ;
   - un radical alkylthio choisi parmi les radicaux méthylthio, éthylthio ou isopropylthio ;
   - un radical alkylamino choisi parmi les radicaux N-méthylamino ou N,N-diméthylamino ;
ou R₄R₅ représentent un radical choisi parmi les radicaux -CH₂CH₂-, -CH₂O-, - CH₂CH₂O-, -CH₂CH₂S-, -CH₂CH₂NR₄- et R₆ est tel que défini précédemment, leurs sels d'addition et les hydrates de ces sels d'addition avec les acides minéraux ou les acides organiques pharmaceutiquement acceptables, ainsi que leurs formes tautomères, les énantiomères, les mélanges d'énantiomères et les stéréoisomères purs ou en mélange racémique ou non.

Le nouveau procédé, objet de la présente invention, s'applique plus particulièrement aux composés de formule (1a), 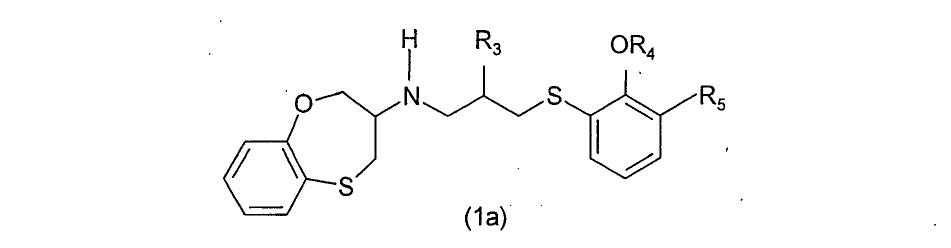 dans laquelle :
R₃ représente un radical alkyle choisi parmi les radicaux méthyle, éthyle ou isopropyle,
R₄ représente un atome d'hydrogène ou un radical méthyle,
R₅ représentent un atome d'hydrogène ou un radical méthyle ;
ou bien R₄R₅ représentent un radical -CH₂CH₂- ;
leurs sels d'addition et les hydrates de ces sels d'addition avec les acides minéraux ou les acides organiques pharmaceutiquement acceptables, ainsi que leurs formes tautomères, les énantiomères, les mélanges d'énantiomères et les stéréoisomères purs ou en mélange racémique ou non.

Dans un mode particulier de réalisation de l'invention, les composés de formule (1a) préférés sont :
N-{3-[(2-Méthoxyphényl)sulfanyl]-2-méthylpropyl}-3,4-dihydro-2H-1,5-benzoxathiépin-3-amine ;
2-{[3-(3,4-Dihydro-2H-1,5-benzoxathiépin-3-ylamino)-2-méthylpropyl]sulfanyl}-6-méthylphénol,
leurs sels d'addition et les hydrates de ces sels d'addition avec les acides minéraux ou les acides organiques pharmaceutiquement acceptables, ainsi que leurs formes tautomères, les énantiomères, les mélanges d'énantiomères et les stéréoisomères purs ou en mélange racémique ou non.

Le nouveau procédé s'applique aux composés de formule (1) et plus particulièrement à ceux de formule (1a) dont l'atome de carbone stéréogénique C(3) du fragment 3,4-dihydro-2H-1,5-benzoxathiépine est de configuration absolue (R) et dont l'atome de carbone stéréogénique qui porte le groupe R₃ est de configuration absolue (S). Les descripteurs (R) et (S), utilisés pour spécifier la configuration absolue des atomes stéréogéniques contenus dans les molécules de formule (1), sont définis tels que dans la règle de priorité de Cahn-Ingold-Prelog (E.L. Eliel and S.H. Wilen Stereochemistry of Organic Compounds, John Wiley & Sons, Inc., chap. 5, 104-12, 1994).

Dans un autre mode particulièrement avantageux de réalisation de l'invention, les composés de formule (1a) sont choisis parmi les stéréoisomères suivants :
(3R)-N-{(2S)-3-[(2-Méthoxyphényl)sulfanyl]-2-méthylpropyl}-3,4-dihydro-2H-1,5-benzoxathiépin-3-amine ;
2-({(2S)-3-[(3R)-3,4-Dihydro-2H-1,5-benzoxathiépin-3-ylamino]-2-méthylpropyl}sulfanyl)-6-méthylphénol,
leurs sels d'addition et les hydrates de ces sels d'addition avec les acides minéraux ou les acides organiques pharmaceutiquement acceptables. Dans la présente invention, les stéréoisomères sont considérés comme purs s'ils sont associés avec moins de 1% d'un autre stéréoisomère ou d'un mélange d'autres stéréoisomères (i.e., excès diastéréoisomérique ≥ 98%, l'actualité chimique 2003, 11/12, 10-4).

Les composés de formule générale (1) sont décrits dans la demande de brevet internationale WO 02/081464 et revendiqués comme utiles dans le traitement de l'angor stable, l'angor instable, l'insuffisance cardiaque, le syndrome du QT long d'origine congénitale, l'infarctus du myocarde et les troubles du rythme cardiaque.

Les composés de formule (1) peuvent être synthétisés selon le procédé décrit dans WO 02/081464. Cependant, le procédé en question présente plusieurs inconvénients :
- il met en oeuvre des matières premières et des réactifs coûteux dont certains sont potentiellement explosifs ;
- alors que le rendement global en composé de formule (1) est faible, la quantité de sous-produits engendrée est considérable ;
- certaines étapes de synthèses ne sont pas reproductibles.
Compte tenu de ces éléments, le procédé décrit dans WO 02/081464 est extrêmement difficile à réaliser, voire irréalisable, au plan semi-industriel ou industriel.

L'objet de la présente invention concerne précisément un nouveau procédé de synthèse des composés de formule (1) et en particulier ceux de formule (1a) qui, contrairement à celui illustré dans WO 02/081464, est tout à fait réalisable à l'échelle semi-industrielle ou industrielle. A ce titre, le procédé de l'invention constitue une amélioration majeure par rapport au procédé antérieur et fournit une voie d'accès particulièrement avantageuse aux composés de formule (1) et en particulier ceux de formule (1a) dont le potentiel thérapeutique est important.

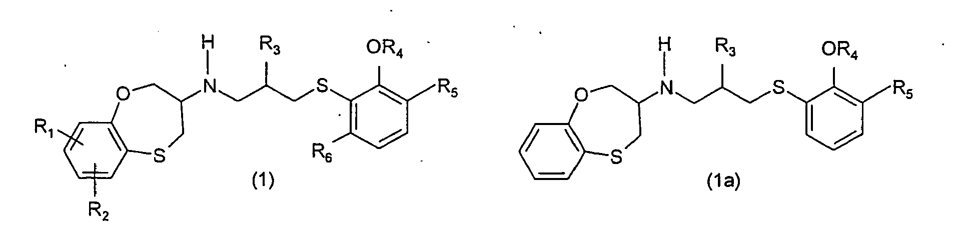

Un premier aspect de l'invention concerne donc l'amélioration du procédé de synthèse des composés de formule (1) et plus particulièrement ceux de formule (1a) par réduction du nouvel intermédiaire de formule (9), respectivement du nouvel intermédiaire (9a) 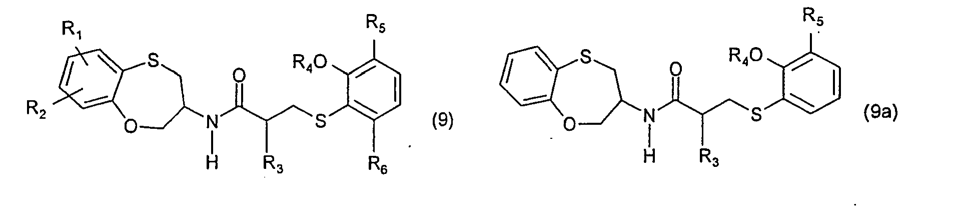 de préférence obtenu par condensation des composés de formule (5) et (7) ou (8) 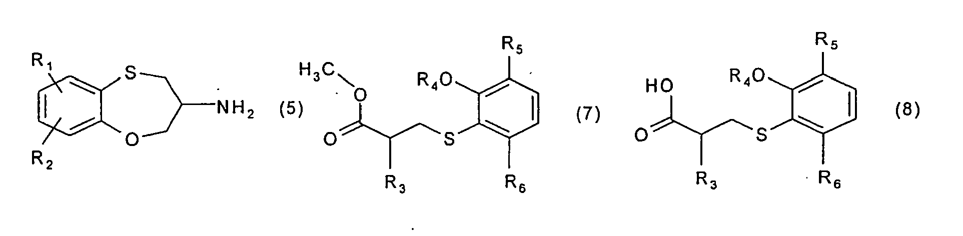 et plus particulièrement par condensation des composés de formule (5) et (7a) ou (8a) 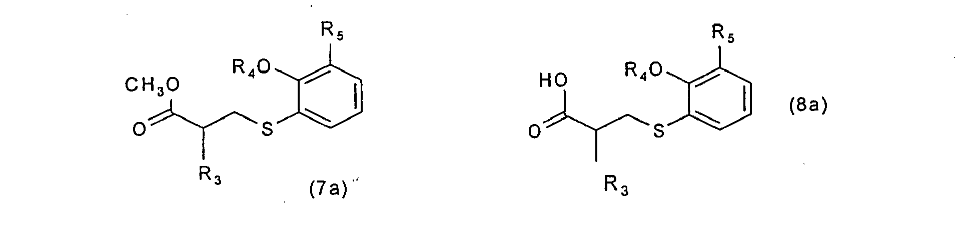 dans lesquelles les radicaux R₁, R₂, R₃, R₄, R₅ et R₆ ont les significations données à propos de la formule (1) ou, de préférence, celles données à propos de la formule (1a).

Le stéréoisomère préféré des composés de formule (1), (1a) et (9), (9a) est, dans tous les cas, celui dont les atomes de carbone stéréogéniques du fragment 3,4-dihydro-2H-1,5-benzoxathiépine et de la chaîne propionamine/propionamide sont de configuration absolue (R) et (S), respectivement.

La présente invention concerne également les nouveaux intermédiares de synthèse de formule (9) et plus particulièrement ceux de formule (9a).

Un deuxième aspect de l'invention comprend l'amélioration du procédé de synthèse des composés de formule (5) 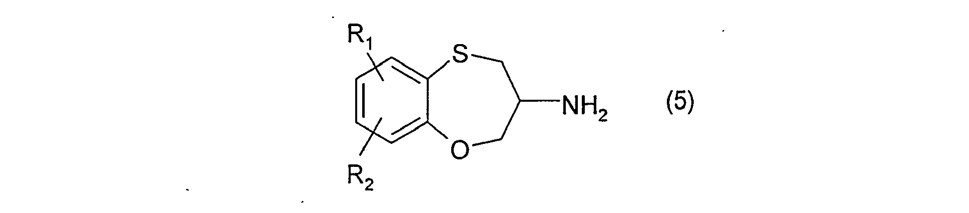 dans laquelle les radicaux R1 et R2 ont les significations données à propos de la formule (1)
selon lequel un composé de formule (2) 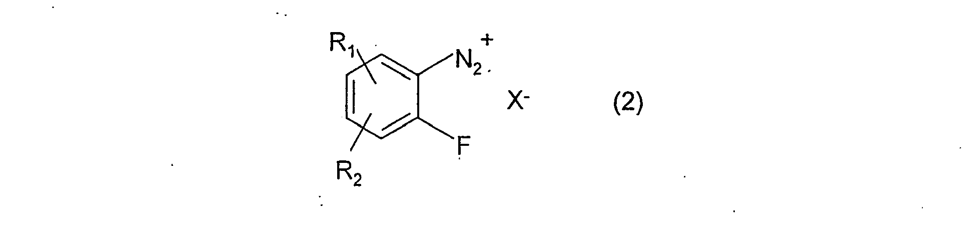 est traité par un ester de cystine, de préférence un ester de L-cystine, pour donner le composé de formule (3) 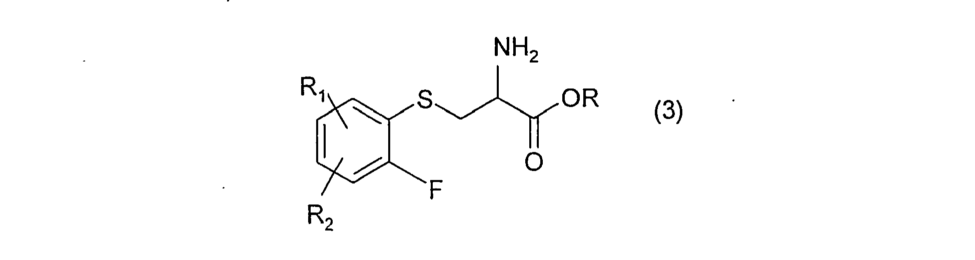 dans laquelle R représente un radical méthyle, qui par réduction au moyen d'un agent ) donneur d'hydrure fournit le composé de formule (4) 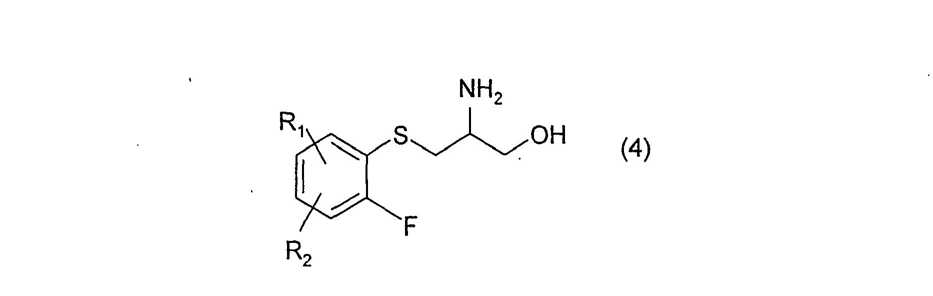 qui est cyclisé, chimiosélectivement, pour donner les composés de formule (5), dans lesquels R₁ et R₂ ont les significations données à propos de la formule (1) ou, de préférence, celles données à propos de la formule (1a). L'énantiomère préféré des composés de formule (3), (4) et (5) est, dans tous les cas, celui dont l'atome de carbone stéréogénique est de configuration absolue (R).
Ce deuxième aspect de l'invention comprend préférentiellement l'amélioration du procédé de synthèse du composé de formule (5a), analogue des composés de formule (5) ci-dessus pour lequel R₁ et R₂ représentent chacun un atome d'hydrogène selon lequel on met en oeuvre les composés de formules (2a), (3a) et (4a), analogues des composés de formule (2), (3) et (4) respectivement et pour lesquels R₁ et R₂ représentent chacun un atome d'hydrogène, dans le procédé décrit ci-dessus pour l'obtention des composés de formule (5).

Un troisième aspect de l'invention inclut le procédé de synthèse des nouveaux intermédiaires de formule (7) et (8) 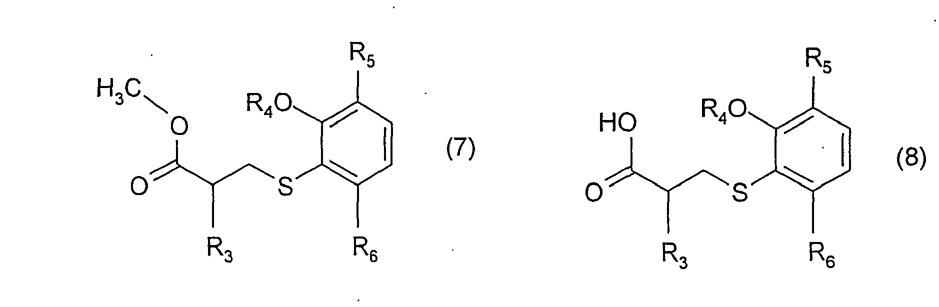 et plus particulièrement des nouveaux intermédiaires de synthèse (7a) et (8a) : 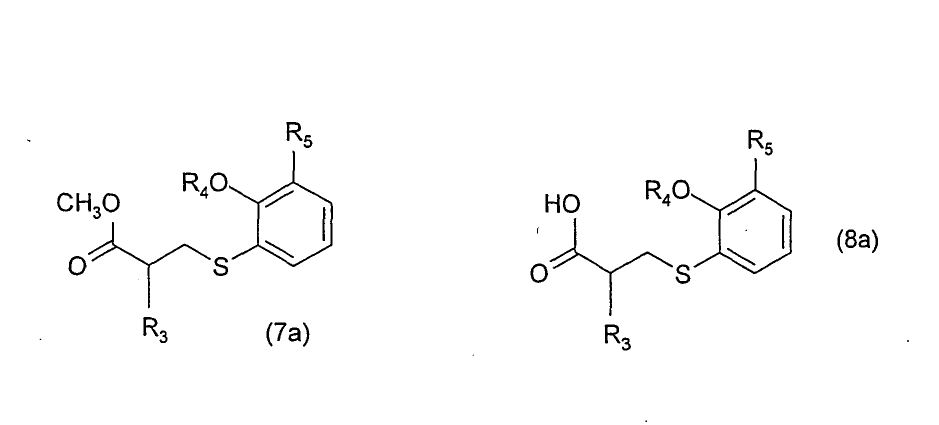 selon lequel le composé du type méthyl 3-[(méthylsulfonyl)oxy]proponoate substitué en position 2, de formule (6) 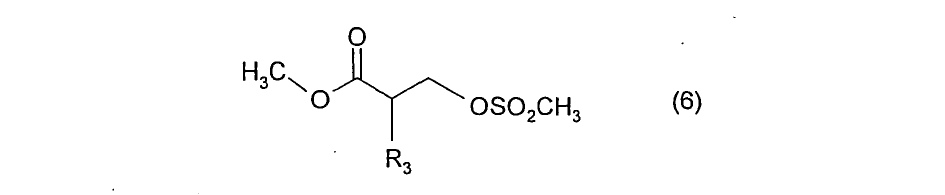 est traité par l'arylthiophénol approprié en présence d'une base organique et/ou minérale pour donner le composé de formule (7), ou plus particulièrement (7a), 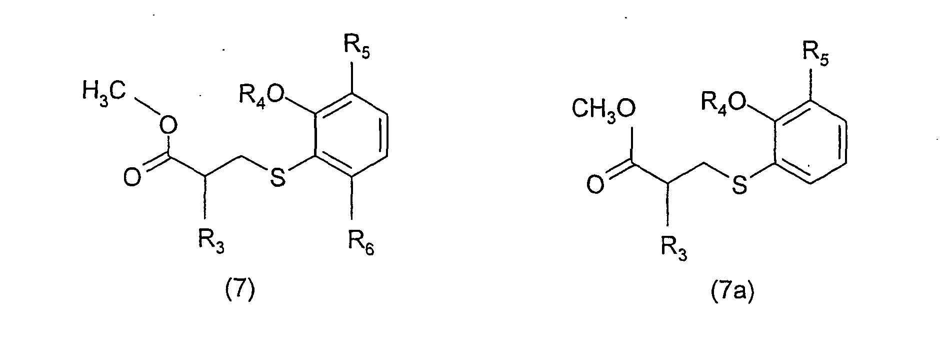 qui peut être hydrolysé en l'acide correspondant de formule (8), ou plus particulièrement (8a) 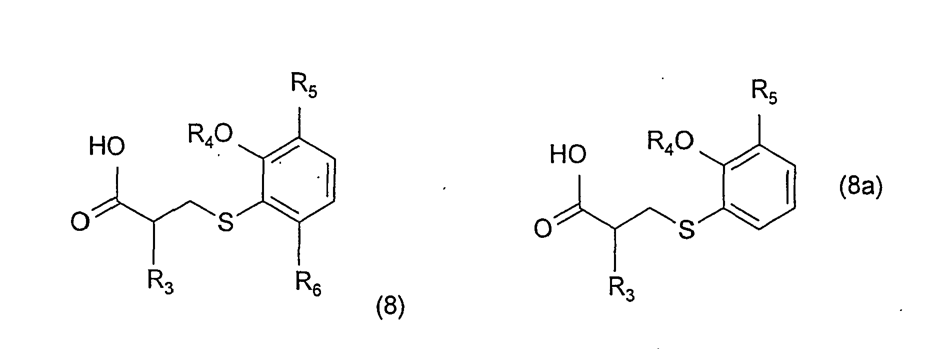 dans lesquels les groupes R₃, R₄, R₅ et R₆ ont les significations données à propos de la formule (1) ou, de préférence, celles données à propos de la formule (1a). L'énantiomère préféré des composés de formule (6) est celui dont l'atome de carbone stéréogénique est de configuration absolue (R) alors que l'énantiomère préféré des composés de formule (7), (8), (7a) et (8a) est celui dont l'atome de carbone stéréogénique est de configuration absolue (S).
Ce troisième aspect de l'invention concerne également les composés de formules (7), et plus particulièrement (7a), à l'exception des composés pour lesquels R₃ représente un radical méthyle tandis que R₄, R₅ et R₆ représentent chacun un atome d'hydrogène.
Le brevet US-3,636,074 divulgue en effet un tel composé. Ce dernier n'est toutefois pas préparé par un procédé analogue à celui qui fait l'objet de la présente invention, et il n'est pas utilisé comme intermédiaire de synthèse dans la préparation de benzoxathiépine.
Dans ce troisième aspect, l'invention concerne aussi les composés de formule (8) ou plus particulièrement les composés de formule (8a).

Le procédé de préparation des composés de formule générale (1) et en particulier ceux de formule (1a), objet de la présente invention est nouveau, plus économique et plus respectueux de l'environnement que le procédé antérieur (WO 02/081464). De plus, contrairement au procédé antérieur, il est réalisable au plan semi-industriel ou industriel. Plus spécifiquement, selon l'état de la technique antérieur (WO 02/081464), les composés de formule (1) et en particulier ceux de formule (1a) provenaient d'une réaction d'amination réductrice entre le composé de formule (5) et l'aldéhyde de formule (A), cf. schéma I. 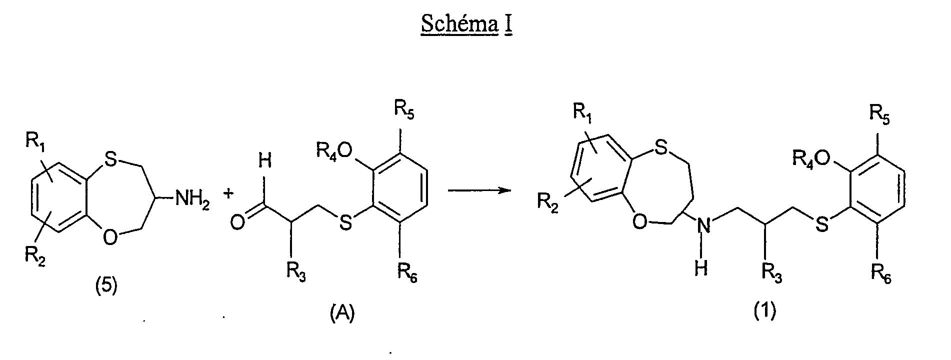 Toutefois, l'aldéhyde de formule (A), en particulier lorsque le radical R₃ représente un groupe méthyle, était instable à la fois chimiquement et stéréochimiquement ; l'instabilité de certains aldéhydes du type de (A) avaient déjà été rapportée e.g., J. Org. Chem. 1987, 52(8), 316-8 ; J. Org. Chem. 2003, 68(12), 5002-5 ; Organic Process Research & Development 2004, 8(1), 92-100. De ce fait, le composé de formule (A) était préparé de façon transitoire à très basse température (< -60°C) puis piégé, *in situ,* au moyen de l'amine (5) et d'un excès de l'agent réducteur. Déjà à l'échelle du laboratoire, le rendement chimique ainsi que la pureté stéréochimique du composé (1) et plus particulièrement ceux de formule (1a) lorsque R₃ = CH₃, obtenus étaient fortement variables et dépendants des conditions opératoires et de la qualité des produits et des réactifs utilisés. La séquence réactionnelle mise en oeuvre n'était donc pas reproductible. Du fait de la nature de certains réactifs utilisés (e.g., chlorure d'oxalyle et dichlorométhane), de la nature de certains des sous-produits formés (e.g., diméthylsulfure) et de la complexité du protocole expérimental (e.g., très basse température et ajouts séquentiels de plusieurs composés parfois en excès) cette séquence réactionnelle était techniquement extrêmement difficile à réaliser, voire irréalisable, au plan semi-industriel ou industriel.
Le nouveau procédé de l'invention (cf. schéma II) présente l'avantage, entre autres, de ne faire intervenir que des composés stables chimiquement et stéréochimiquement. 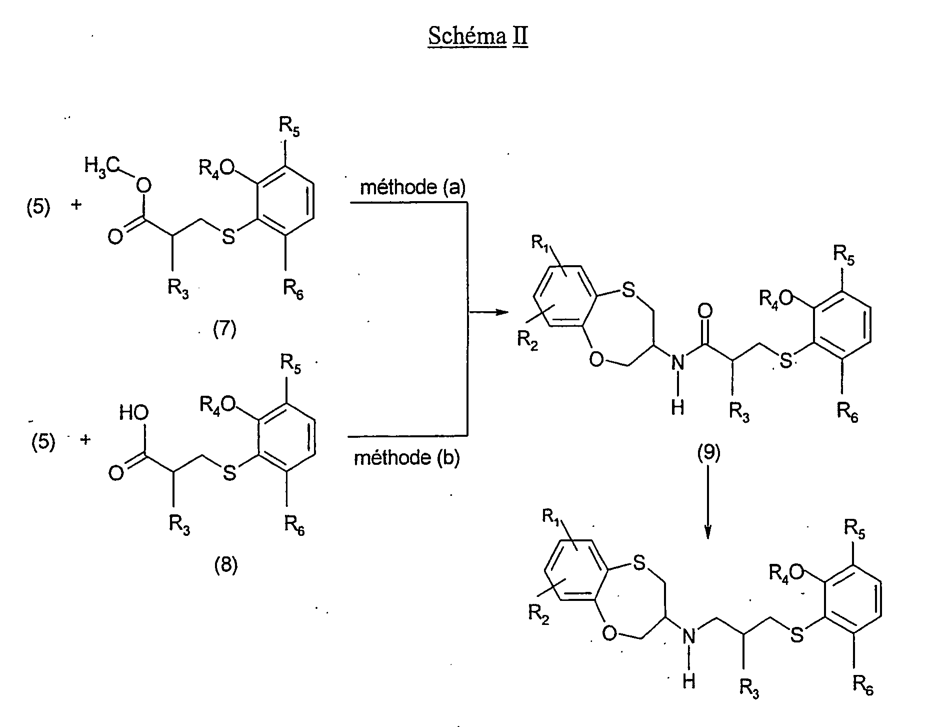 Ainsi:
- selon la méthode (a), l'amine de formule (5) et l'ester de formule (7) sont condensés en présence d'un dérivé d'alkylaluminium tel que, par exemple, de l'hydrure de diisobutylaluminium selon une méthode analogue à celle rapportée dans Tetrahedron Lett. 2001, 42(51), 9039-41.
- Selon la méthode (b), l'amine de formule (5) et l'acide de formule (8) sont condensés au moyen d'un agent de couplage tel que, par exemple, le carbonyl diimidazole selon une méthode analogue à celle décrite dans J. Org. Chem. 2003, 68(7), 2633-38.
L'amide (9), formé par l'une des méthodes (a) ou (b), est généralement un composé cristallin, stable chimiquement et stéréochimiquement qui peut être purifié, si on le désire, par recristallisation. Le composé de formule (9) est ensuite réduit, sans diminution de la pureté stéréochimique, en l'amine de formule (1) au moyen d'un complexe de borane tel que, par exemple, le complexe borane-THF. Le composé de formule (1) peut être ensuite, si on le souhaite, salifié au moyen d'un acide organique ou inorganique pharmaceutiquement acceptable. Le nouveau procédé est donc reproductible, robuste et réalisable au plan semi- ou industriel.
Un aspect additionnel de la présente invention consiste en l'amélioration du procédé de synthèse de l'amine de formule (5).
Dans la demande de brevet WO 02/081464, la synthèse du composé (5) utilisait comme matières premières un 2-hydroxy-benzenethiol approprié et la N-Boc-L-sérine. La synthèse du composé (5), selon la voie proposée, comprenait alors cinq étapes dont deux faisaient appel à des réactions de type Mitsunobu et une à l'hydrolyse d'un groupement protecteur. Or, les deux réactions de type Mitsunobu engendraient une quantité de sous-produits (i.e., triphénylphosphine oxyde et hydrazinodicarboxylate d'alkyle) très supérieure à celle du produit cible (> 400% en poids), difficiles à séparer de ce dernier et non recyclables. De plus, un des réactifs utilisé (i.e., diazodicarboxylate d'alkyle) est connu pour son instabilité. La déprotection de la fonction amine primaire, en dernière étape, occasionnait aussi une perte de poids importante (> 50%) en composé de formule (5). Enfin, les produits de départ tels que les 2-hydroxy-benzenethiols sont, généralement, fortement odorants, difficiles à obtenir et/ou à conserver purs car facilement oxydables. En termes d'économie d'atomes, de traitement des effluents et donc de prix de revient, la préparation de l'amine (5) selon le procédé décrit dans la demande de brevet WO 02/081464, présentait un bilan très défavorable. Comparativement, la préparation de l'amine de formule (5) selon le procédé de l'invention, est beaucoup plus avantageuse (cf. schéma III). 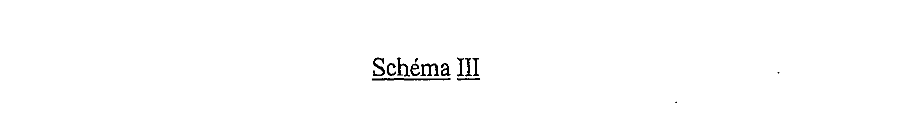 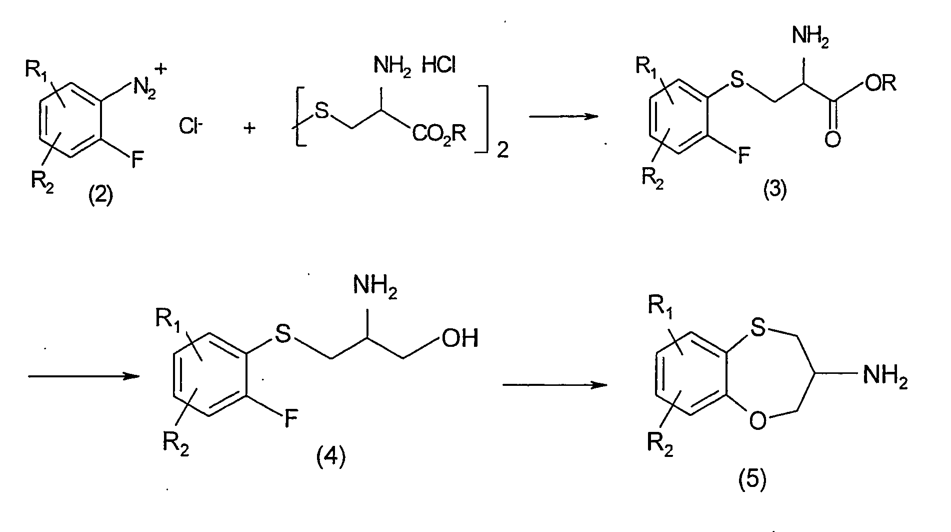 Ainsi, la réaction du diméthylester de L-cystine [32854-09-4] sur un sel de diazonium approprié de formule (2) selon une méthode analogue à celle décrite dans le brevet US 5,599,992, conduit au composé de formule (3). Cette méthodologie, bien que classique en chimie organique (e.g., WO 00/39079), n'a jamais été appliquée dans le cas d'un diester de cystine. L'amino-ester de formule (3) n'est pas purifié mais réduit en l'amino-alcool de formule (4) au moyen d'un agent donneur d'hydrure tel que, par exemple, l'hydrure de lithium-aluminium. Le composé (4) n'est pas purifié mais cyclisé, chimiosélectivement, en l'amine de formule (5). La dite amine (5) peut être soit engagée en l'état dans la réaction suivante, soit salifiée, par exemple sous forme d'un chlorhydrate. L'amine de formule (5) ou son chlorhydrate peut être utilisé dans la réaction de couplage avec les composés de formule (7) ou (8), cf. exemple 4, méthode (c). L'accès au composé de formule (5) selon l'invention, comporte donc moins d'étapes et procède avec un meilleur rendement que celui proposé dans WO 02/081464. Le nouveau procédé permet, également, d'éviter les inconvénients inhérents au procédé antérieur tels que, l'emploi de matières premières difficilement accessibles, de réactifs coûteux, la formation de quantités considérables de sous-produits et l'utilisation de groupement protecteur.

Un autre aspect additionnel de l'invention inclut le procédé de préparation des nouveaux intermédiaires de formule (7) et (8), cf. schéma IV. 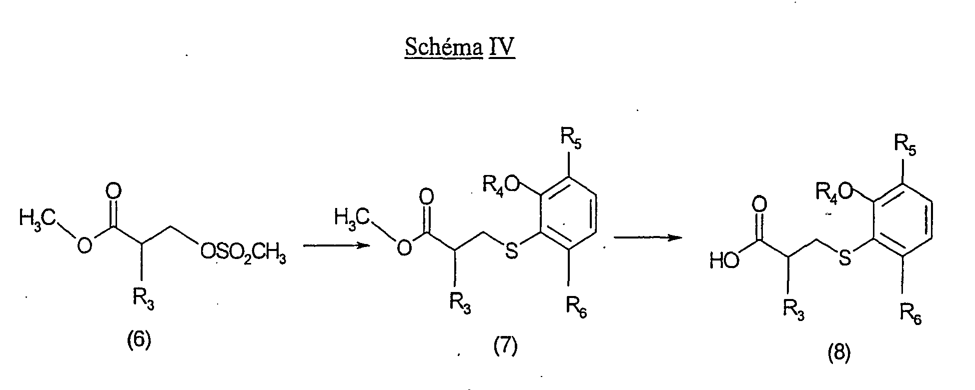 Ainsi, la fonction alcool du méthyl 3-hydroxy-2-alkyl-propanoate est d'abord activée sous la forme d'un mésylate de formule (6) puis déplacée par le thiophénol approprié pour donner le composé de formule (7). Le composé de formule (7) peut être :
- soit engagé directement dans la réaction de condensation avec l'amine (5), méthode (a), schéma II ;
- soit hydrolysé en l'acide correspondant dé formule (8) puis engagé dans la réaction de condensation avec l'amine (5), méthode (b), schéma II.
Le dit acide (8) est cristallin, stable chimiquement et stéréochimiquement et peut être, si on le désire, purifié par recristallisation ce qui, dans certains cas particuliers, peut représenter un avantage.
Les exemples suivants illustrent l'invention sans toutefois en limiter la portée.

### Exemple 1 : (3R)-3,4-dihydro-2H-1,5-benzoxathiépin-3-amine (5a-1) [470662-82-9]

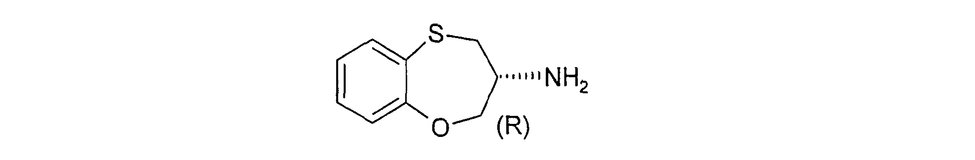

### Etape 1 : 2-Fluorobenzènediazonium (2a-1) [45660-02-4]

Dans un ballon, on ajoute 4,1 mL (48,8 mmoles) d'acide chlorhydrique à 36% à une solution de 2,36 mL (24,4 mmoles) de fluoroaniline dans 15 mL d'eau distillée. Le milieu réactionnel est porté à 60 °C pendant 15 minutes puis refroidi à 0 °C. On ajoute alors goutte à goutte une solution de 1,68 g (24,4 mmoles) de nitrite de sodium dissous dans 10 mL d'eau distillée. La température du mélange est maintenue entre 2-5°C pendant toute la durée de l'addition et le mélange agité pendant 15 minutes après la fin de l'addition. La solution aqueuse homogène contenant le composé (2a-1) est utilisée directement dans l'étape suivante.

### Etape 2 : Méthyl S-(2-fluorophényl)-L-cystéinate (3a-1)

A une solution de 4,16 g (12,2 mmoles) de dichlorhydrate de diméthylester de L-cystine et 328 mg (2,44 mmoles) de CuCl₂ dans 19 mL d'eau distillée maintenue à 50-55 °C, on ajoute goutte à goutte (environ 30 minutes) la solution aqueuse contenant le sel de diazonium (2a-1) obtenue à l'étape 1. Le milieu réactionnel, hétérogène, est agité pendant 5 minutes après la fin du dégagement gazeux puis refroidi à température ambiante et lavé à l'éther éthylique. La phase aqueuse est neutralisée par ajout d'une solution aqueuse d'ammoniaque à 32% (5,6 ml, 94 mmoles) puis extraite à l'acétate d'éthyle. Les phases organiques réunies sont lavées par une solution de bisulfite de sodium à 10% puis par de la saumure, séchées sur sulfate de sodium, filtrées et concentrées sous pression réduite. On obtient une huile brune (3,7 g) que l'on engage sans autre purification dans l'étape suivante.
¹H RMN (CDCl₃) δ : 1,69 (ls, 2H échangeables); 3,15 (dd, 1H) ; 3,30 (dd, 1H) ; 3,60 (s, 3H) ; 3,62 (m, 1H) ; 7,08 (d, 1H) ; 7,12 (d, 1H) ; 7,26 (m, 1H) ; 7,46 (dd, 1H) ;
IR (lames) ν : 1739 cm⁻¹.

### Etape 3 : (2R)-2-amino-3-[(2-fluorophényl)sulfanyl]propan-1-ol (4a-1)

Dans un ballon maintenu sous atmosphère inerte, on introduit 23,9 mL (23,9 mmoles) d'hydrure de lithium et d'aluminium (1M dans le THF). La solution est refroidie à 0 °C puis on ajoute 4,57 g (19,9 mmoles) de méthyl S-(2-fluorophényl)-L-cystéinate (3a-1) dilués dans 21 mL de THF anhydre. La solution est agitée à 0 °C pendant 1 heure puis à température ambiante pendant trente minutes. Le milieu réactionnel est hydrolysé par ajouts successifs de 2,7 mL d'eau, 0,9 mL de soude à 15% dans l'eau puis 0,9 mL d'eau. La suspension est agitée pendant trente minutes à température ambiante en présence de sulfate de sodium puis filtrée sur filtre de silice. Le précipité est lavé avec du THF puis le filtrat concentré sous pression réduite. On obtient une huile brune (3,56 g) que l'on engage sans autre purification dans l'étape suivante.
¹H RMN (CDCl₃) δ : 1,45 (ls, 2H échangeables) ; 1,86 (m, 1H) ; 2,79 (dd, 1H) ; 3,08 (dd, 1H) ; 3,43 (dd, 1H) ; 3,64 (dd, 1H) ; 3,75 (m, 1H) ; 7,06 (d, 1H) ; 7,10 (d, 1H) ; 7,25 (m, 1H) ; 7,42 (dd, 1H).

### Etape 4 : (3R)-3,4-dihydro-2H-1,5-benzoxathiépin-3-amine (5a-1) [470662-82-9]

Dans un ballon maintenu sous atmosphère inerte, on introduit 3,55 g (17,6 mmoles) de (2R)-2-amino-3-[(2-fluorophényl)sulfanyl]propan-1-ol (4a-1) et 71 mL de dioxane. La solution est refroidie à 0 °C puis on ajoute par portions 9,90 g (88,2 mmoles) de *tert-*butylate de potassium. On agite à température ambiante pendant 12 heures. Le mélange est ensuite concentré sous pression réduite et le résidu repris par de l'eau puis extrait par de l'acétate d'éthyle. Les phases organiques réunies sont lavées par de l'eau, de la saumure, séchées sur sulfate de sodium, filtrées et concentrées sous pression réduite. L'huile brune ainsi obtenue est reprise par une solution de HCl 2,2N dans l'éthanol (10,9 mL). Le mélange est concentré sous pression réduite et le chlorhydrate du composé du titre est précipité par addition d'éther isopropylique. Le solide est filtré, lavé avec de l'éther isopropylique puis séché sous vide. On obtient le chlorhydrate du composé du titre sous forme d'un solide crème (2,73 g, 12,5 mmoles).
Rendement sur 4 étapes : 51 % ;
F = 235 °C;
[α] = + 48,9 (c = 0,350, méthanol) ;
¹H RMN (CDCl₃) δ : 1,69 (ls, 2H échangeables) ; 2,77 (dd, 1H) ; 3,17 (dd, 1H) ; 3,42 (m, 1H) ; 4,07 (dd, 1H) ; 4,12 (dd, 1H) ; 6,96 (m, 2H) ; 7,14 (dt, 1H) ; 7,36 (dd, 1H). ¹H RMN (DMSOd₆) δ : 3,12 (dd, 1H) ; 3,21 (dd, 1H) ; 3,81 (m, 1H) ; 4,21 (dd, 1H) ; 4,31 (dd, 1H) ; 7,09 (m, 2H) ; 7,28 (td, 1H) ; 7,45 (dd,1H) ; 8,64 (ls, 3H échangeables).

### Exemple 2 : Méthyl (2S)-3-[(2-méthoxyphényl)sulfanyl]-2-méthylpropanoate (7a-1)

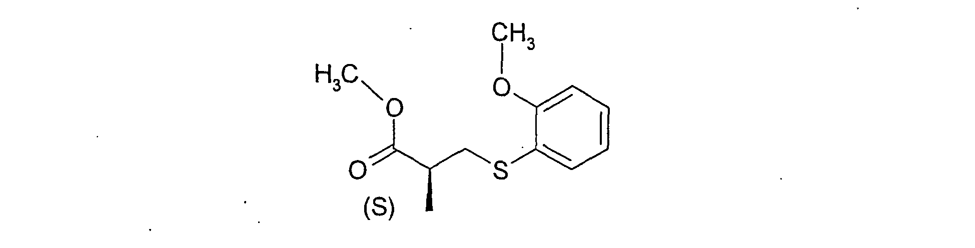

### Etape 1 : Méthyl (2R)-2-méthyl-3-[(méthylsulfonyl)oxy]propanoate (6a-1)

Dans un ballon maintenu sous atmosphère inerte, on introduit 4 g (33,9 mmoles) de méthyl (2R)-3-hydroxy-2-méthylpropanoate, 5,43 mL (38,9 mmoles) de triéthylamine, 4,68 g (33,9 mmoles) de carbonate de potassium et 40 mL de THF anhydre. A cette solution maintenue à 0 °C, on ajoute 2,88 mL (37,2 mmoles) de chlorure de méthanesulfonyle et le mélange est agité à température ambiante pendant 12 heures. Le précipité blanc formé est filtré, rincé par du THF et le filtrat concentré sous pression réduite. Le résidu est repris par de l'éther éthylique et la phase organique lavée à l'eau jusqu'à neutralité de l'eau de lavage. La phase organique est lavée par de la saumure, séchée sur sulfate de sodium, filtrée et concentrée sous pression réduite. On obtient le composé du titre sous la forme d'une huile incolore (5,13 g) que l'on engage sans autre purification dans l'étape suivante.
¹H RMN (CDCl₃) δ : 1,26 (d, 3H) ; 2,90 (m, 1H) ; 3,03 (s, 3H) ; 3,74 (s, 3H) ; 4,27 (dd, 1H) ; 4,38 (dd, 1H) ;
IR (lames) ν : 966, 1736 cm⁻¹.

### Etape 2 : Méthyl (2S)-3-[(2-méthoxyphényl)sulfanyl]-2-méthylpropanoate (7a-1)

Dans un ballon maintenu sous atmosphère inerte, on introduit 4,72 mL (38,8 mmoles) de 2-méthoxythiophénol, 10,72 g (77,6 mmoles) de carbonate de potassium, 5,13 g de méthyl (2R)-2-méthyl-3-[(méthylsulfonyl)oxy]propanoate (6a-1) dilués et 100 mL de THF anhydre puis 329 mg (0,97 mmole) d'hydrogénosulfate de tétrabutylammonium. Le milieu réactionnel est porté à 60 °C pendant 24 h sous agitation vigoureuse puis concentré sous pression réduite. Le résidu est repris par de l'eau et est extrait par de l'éther éthylique. Les phases organiques réunies sont lavées par une solution de soude 1N, par de l'eau, de la saumure puis séchées sur sulfate de sodium, filtrées et concentrées sous pression réduite. Le résidu est repris dans du méthanol et concentré puis repris dans du méthanol (10ml / 1g de résidu). Le bis(methoxyphenyl) disulfide [13920-94-0] qui précipite lentement est éliminé par filtration. Le filtrat est évaporé sous pression réduite pour donner le composé du titre sous la forme d'une huile jaune pale (6.4 g, 26.6 mmoles).
Rendement sur 2 étapes : 69% ;
[α] = - 71,6 (c = 0,173, méthanol) ;
¹H RMN (CDCl₃₎ δ : 1,28 (d, 3H) ; 2,67 (m, 1H) ; 2,88 (dd, 1H) ; 3,25 (dd, 1H) ; 3,66 (s, 3H) ; 3,89 (s, 3H) ; 6,86 (d, 1H) ; 6,91 (dd, 1H) ; 7,21 (dd, 1H); 7,32 (d, 1H) ;
IR (lames) ν: 1732 cm⁻¹ ;
HPLC, colonne Chiralcel OD (hexane / isopropanol 98 : 2, 1 mL/min) : temps de rétention Tr = 13.28 min.

### Etape 3 : Acide (2S)-3-[(2-méthoxyphényl)sulfanyl]-2-méthylpropanoïque (8a-1)

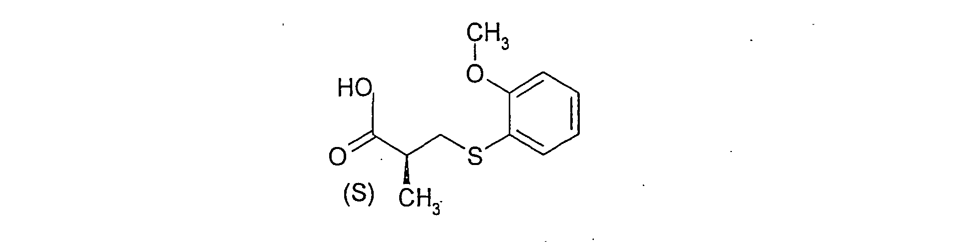

Dans un ballon, on introduit 7,11 g (29,6 mmoles) de méthyl (2S)-3-[(2-méthoxyphényl)sulfanyl]-2-méthylpropanoate (7a-1) et 35,5 mL de THF. On ajoute 6,21 g (0,148 mole) de LiOH•H₂O dissous dans 35,5 mL d'eau. Le mélange est agité à 50 °C pendant 12 heures puis concentré sous pression réduite. Le résidu est repris par de l'eau et extrait par du toluène. La phase aqueuse est acidifiée (pH = 2) par ajout d'acide chlorhydrique à 36% puis extraite à l'acétate d'éthyle. Les phases organiques réunies sont lavées par de la saumure, séchées sur sulfate de sodium et concentrées sous pression réduite. On obtient le composé du titre sous la forme d'un solide blanc (5 g, 22,1 mmoles) qui peut être, si on le désire, recristallisé dans l'éther isopropylique.
Rendement sur 3 étapes : 65% ;
F = 103 °C ;
[α] = - 41,5 (c = 0,492, méthanol) ;
¹H RMN (CDCl₃) δ : 1,31 (d, 3H) ; 2,69 (m, 1H) ; 2,88 (dd, 1H) ; 3,27 (dd, 1H) ; 6,86 (d, 1H) ; 6,91 (dd, 1H) ; 7,22 (dd, 1H) ; 7,34 (d, 1H) ; 10,85 (ls, 1H échangeable) ;
IR (KBr) ν: 1720, 3114 cm⁻¹ ;
HPLC, colonne Chiralcel OD (hexane / éthanol / TFA 95 : 5 : 0.5, 1 mL/min) : Tr = 14.57 min.

| Analyse C₁₁H₁₄O₃S : | | |
|---|---|---|
| Calc.% : | C58,38 | H6,24 |
| Tr. : | C58,41 | H6,32 |

### Exemple 3 : Méthyl (2R)-3-[(2-méthoxyphényl)sulfanyl]-2-méthylpropanoate (7a-2)

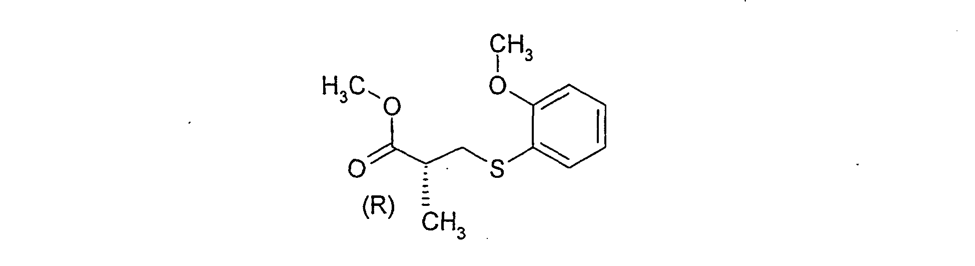

### Etape 1 : Méthyl (2R)-3-[(2-méthoxyphényl)sulfanyl]-2-méthylpropanoate (7a-2)

En procédant comme dans l'étape 2 de l'exemple 2 mais en utilisant le méthyl (2S)-2-méthyl-3-[(méthylsulfonyl)oxy]propanoate [142402-78-6] à la place du méthyl (2R)-2-méthyl-3-[(méthylsulfonyl)oxy]propanoate (6a-1) on obtient le composé du titre.
[α] = + 67,1 (c = 0,368, méthanol) ;
HPLC, colonne Chiralcel OD (hexane / isopropanol 98 : 2, 1 mL/min) : Tr = 16.47 min.

### Etape 2 : Acide (2R)-3-[(2-méthoxyphényl)sulfanyl]-2-méthylpropanoïque (8a-2)

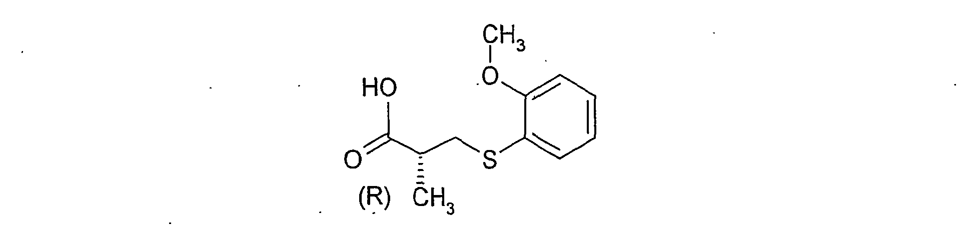

En procédant comme dans l'étape 3 de l'exemple 2 mais en utilisant le méthyl (2R)-3-[(2-méthoxyphényl)sulfanyl]-2-méthylpropanoate (7a-2) à la place du méthyl (2S)-3-[(2-méthoxyphényl)sulfanyl]-2-méthylpropanoate (7a-1) on obtient le composé du titre.
[α] = + 43,3 (c = 0,455, méthanol) ;
HPLC, colonne Chiralcel OD (hexane / éthanol / TFA 95 : 5 : 0.5, 1 mL/min) : Tr = 18.09 min.

### Exemple 4 : (2S)-N-[(3R)-3,4-dihydro-2H-1,5-benzoxathiépin-3-yl]-3-[(2-méthoxyphényl)sulfanyl]-2-méthylpropanamide (9a-1)

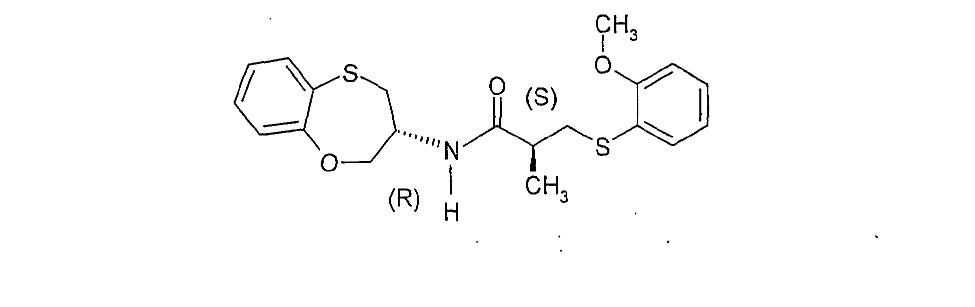

**Méthode (a) :** Le chlorhydrate de (3R)-3,4-dihydro-2H-1,5-benzoxathiépin-3-amine (1,5 g, 6.89 mmoles) est ajouté à température ambiante à une solution de NaOH 10N (10 ml). Le mélange est ensuite extrait par de l'éther éthylique, la phase organique lavée à l'eau salée, séchée sur sulfate de sodium puis concentrée sous pression réduite. Le résidu est repris dans du dichlorométhane ou du toluène et concentrée sous vide. L'amine (5a-1) est récupérée sous la forme d'une huile incolore (1,1 g, 6,07 mmoles) utilisée dans l'étape suivante sans autre purification.
Rendement : 88% ;
Dans un ballon maintenu sous atmosphère inerte, on introduit 1,1 g de (3R)-3,4-dihydro-2H-1,5-benzoxathiépin-3-amine (6,07 mmoles) et 10 ml de THF. A cette solution refroidie à 0°C, on ajoute goutte à goutte un solution d'hydrure de diisopropylaluminium 1M dans le THF (6,7 ml, 6.7 mmoles). Lorsque le dégagement gazeux à cessé, le mélange réactionnel est agité à température ambiante pendant 1 heure puis refroidi à 0°C. On ajoute le méthyl (2S)-3-[(2-méthoxyphényl)sulfanyl]-2-méthylpropanoate (7a-1), 1,6 g, 6,67 mmoles, puis on chauffe le mélange à 50°C pendant 12 heures. Le mélange réactionnel est versé dans une solution aqueuse saturée en sel de Rochelle (70 ml) et le mélange agité jusqu'à ce qu'il devienne limpide. On extrait le mélange avec de l'acétate d'éthyle, la phases organiques réunies sont lavées à l'eau, à l'eau salée, séchées sur sulfate de sodium puis filtrées et concentrées sous pression réduite. Le résidu est repris avec de l'éther diisopropylique, concentré puis repris dans de l'éther diisopropylique. Le composé du titre cristallise alors lentement. Le précipité est filtré, lavé avec de l'éther diisopropylique froid et séché sous vide. On obtient le composé du titre sous la forme d'un solide blanc (1,3 g, 3,34 mmoles).
Rendement : 55% ;

**Méthode (b) :** Dans un ballon maintenu sous atmosphère inerte, on introduit 200 mg (0,884 mmole) d'acide (2S)-3-[(2-méthoxyphényl)sulfanyl]-2-méthylpropanoïque (8a-1) et 2 mL de THF anhydre. On ajoute alors 148 mg (0,91 mmole) de N,N'-carbonyldiimidazole par fractions. Le mélange est agité à température ambiante pendant 3 heures puis on ajoute 160 mg (0,884 mmole) de (3R)-3,4-dihydro-2H-1,5-benzoxathiépin-3-amine (5a-1) dissous dans 1,5 mL de THF anhydre. La solution est agitée à température ambiante pendant 12 heures. Le milieu est hydrolysé par 2 mL d'une solution H₃PO₄ 3M. La phase aqueuse est extraite par de l'acétate d'éthyle. Les phases organiques réunies sont lavées par une solution H₃PO₄ 1M, une solution saturée en NaHCO₃, de l'eau et de la saumure puis séchées sur sulfate de sodium, filtrées et concentrées sous pression réduite. On obtient le composé du titre sous la forme d'un solide blanc (264 mg, 0,678 mmole).
Rendement : 77% ;

**Méthode (c) :** Dans un ballon maintenu sous atmosphère inerte, on introduit 200 mg (0,884 mmole) d'acide (2S)-3-[(2-méthoxyphényl)sulfanyl]-2-méthylpropanoïque (8a-1) et 3,5 mL de THF anhydre. On ajoute alors 148 mg (0,91 mmole) de N,N'-carbonyldiimidazole par fractions. Le mélange est agité à température ambiante pendant 3 heures puis on ajoute 193 mg (0,884 mmole) de chlorhydrate de (3R)-3,4-dihydro-2H-1,5-benzoxathiépin-3-amine et 0,219 mL (1,33 mmoles) de diisopropyléthylamine. La solution est agitée à température ambiante pendant 12 heures. Le mélange réactionnel est traité comme dans la méthode (b) pour donner le composé du titre (250 mg, 0,642 mmole).
Rendement : 73% ;
F =102 °C ;
[α] = - 46,14 (c = 0,485, méthanol) ;
¹H RMN (CDCl₃) δ :1,28 (d, 3H) ; 2,50 (m, 1H) ; 2,93 (m, 2H) ; 3,03 (dd, 1H) ; 3,22 (dd, 1H) ; 3,89 (s, 3H) ; 3,92 (m, 1H) ; 4,32 (dd, 1H) ; 4,60 (m, 1H) ; 6,77 (1d, 1H) ; 6,95 (m, 4H) ; 7,20 (m, 2H) ; 7,37 (d, 1H) ; 7,44 (d, 1H) ;
IR (KBr) ν : 1640, 3278 cm⁻¹ ;

### Exemple 5 : (3R)-N-{(2S)-3-[(2-méthoxyphényl)sulfanyl]-2-méthylpropyl}-3,4-dihydro-2H-1,5-benzoxathiépin-3-amine (1a-1) [470454-73-0]

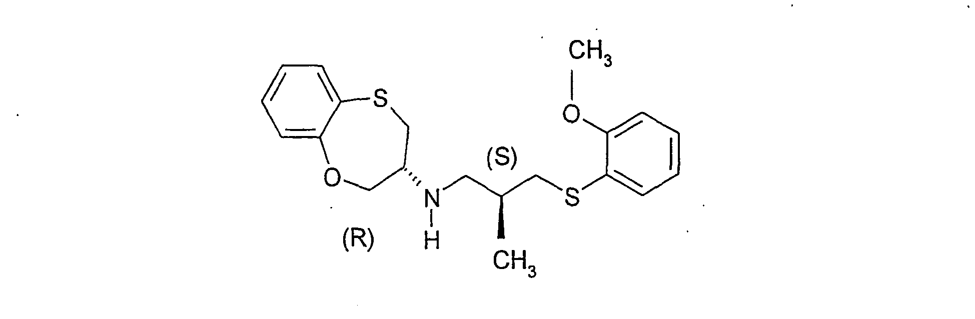

Dans un ballon maintenu sous atmosphère inerte, on introduit 250 mg (0,642 mmole) de (2S)-N-[(3R)-3,4-dihydro-2H-1,5-benzoxathiépin-3-yl]-3-[(2-méthoxyphényl)sulfanyl]-2-méthylpropanamide (9a-1) et 7,5 mL de THF anhydre. On ajoute goutte à goutte 3,21 mL (3,21 mmoles) de BH₃•THF 1M puis on chauffe la solution au reflux pendant 1h30. Le mélange est refroidi, concentré sous pression réduite, repris par 20 mL de méthanol puis acidifié par de l'acide chlorhydrique à 36%. Le milieu est porté 4 heures à reflux puis agité 12 heures à température ambiante. Le mélange réactionnel est concentré sous pression réduite puis rendu basique (pH = 10) par ajout de soude concentrée. La phase aqueuse est extraite au dichlorométhane, les phases organiques réunies sont lavées par de la saumure, séchées sur sulfate de sodium, filtrées et concentrées sous pression réduite. On obtient le composé du titre sous la forme d'une huile incolore (232 mg, 0,618 mmole).
Rendement : 96% ;
¹H RMN (CDCl₃) δ : 1,10 (d, 3H) ; 1,66 (ls, 1H échangeable) ; 1,92 (m, 1H) ; 2,63 (dd, 1H) ; 2,77 (dd, 1H) ; 2,79 (dd, 1H) ; 2,94 (dd, 1H) ; 3,08 (dd, 1H) ; 3,10 (dd, 1H) ; 3,14 (m, 1H) ; 3,90 (s, 3H) ; 4,07 (dd, 1H) ; 4,24 (dd , 1H) ; 6,84 (d, 1H) ; 6,93 (d, 1H) ; 7,00 (m, 2H) ; 7,15 (m, 2H) ; 7,33 (m, 2H) ;
HPLC, Colonne Chiracel OJ, thermostatée à 40°C (méthanol / éthanol / diéthylamine 49.95 : 49.95 : 0.1, 1ml/min) : Tr = 25.11 min ;
La pureté optique du composé (1a-1), calculée à partir des rapports des aires sous la courbe des 4 stéréoisomères, est de 98.5% (excès diastéréoisomérique = 97%).

### Exemple 6 : (3R)-N-{(2R)-3-[(2-méthoxyphényl)sulfanyl]-2-méthylpropyl}-3,4-dihydro-2H-1,5-benzoxathiépin-3-amine (1a-2) [470454-75-2]

HPLC, Colonne Chiracel OJ, thermostatée à 40°C (méthanol / éthanol / diéthylamine 49.95 : 49.95 : 0.1, 1ml/min) : Tr = 16.89 min.

### Exemple 7 : (3S)-N-{(2S)-3-[(2-méthoxyphényl)sulfanyl]-2-méthylpropyl}-3,4-dihydro-2H-1,5-benzoxathiépin-3-amine (1a-3)

HPLC, Colonne Chiracel OJ, thermostatée à 40°C (méthanol / éthanol / diéthylamine 49.95 : 49.95 : 0.1, 1ml/min) : Tr = 20.09 min.

### Exemple 8 : (3S)-N-{(2R)-3-[(2-méthoxyphényl)sulfanyl]-2-méthylpropyl}-3,4-dihydro-2H-1,5-benzoxathiépin-3-amine (1a-4)

HPLC, Colonne Chiracel OJ, thermostatée à 40°C (méthanol / éthanol / diéthylamine 49.95 : 49.95 : 0.1, 1ml/min) : Tr = 14.94 min.

### Exemple 9 : (3R)-N-{(2S)-3-[(2-méthoxyphényl)sulfanyl]-2-méthylpropyl}-3,4-dihydro-2H-1,5-benzoxathiépin-3-amine, bromhydrate (1a-1b)

A une suspension de (3R)-N-{(2S)-3-[(2-méthoxyphényl)sulfanyl]-2-méthylpropyl}-3,4-dihydro-2H-1,5-benzoxathiépin-3-amine (1 g, 2,66 mmoles) dans 20 ml d'eau on ajoute 0,35 mL (3,0 mmoles) d'une solution de HBr à 48% dans l'eau. Le mélange est chauffé et de l'acétone ajoutée jusqu'à obtenir une solution homogène. Le bromhydrate du composé du titre qui cristallise au cours du refroidissement de la solution est filtré, lavé avec de l'acétone puis séché sous vide. On obtient le composé du titre sous la forme d'un solide blanc (0,87 g, 1,91 mmoles).
Rendement : 72% ;
F=129-131°C;
[α] = -9,6 (c = 0,187, méthanol) ;
¹H RMN (DMSO-d₆) δ 1.12 (d, 3H), 2.19 (m, 1H), 2.82 (dd, 1H), 3.05 (lm, 1H), 3.12 (dd, 1H), 3.23 (lm, 1H), 3.32 (m, 2H), 3.82 (s, 3H), 3.90 (ls, 1H), 4.39 (dd, 1H), 4.52 (dd, 1H), 6.98 (m, 2H), 7.07 (m, 2H), 7.23 (m, 3H), 7.40 (dd, 1H), 8.88 (ls, 2H échangeables) ;

| Analyse : C₂₀H₂₆BrNO₂S₂ : | | | |
|---|---|---|---|
| Calc.% : | C52,63 | H5,74 | N3,07 |
| Tr. : | C52,26 | H5,66 | N3,21 |

La pureté optique du composé (1a-1b), calculée à partir des rapports des aires sous la courbe est de 99.2% (excès diastéréoisomérique = 98.4%).

## Revendications

1. Procédé de préparation des dérivés de benzoxathiépines de formule (1) 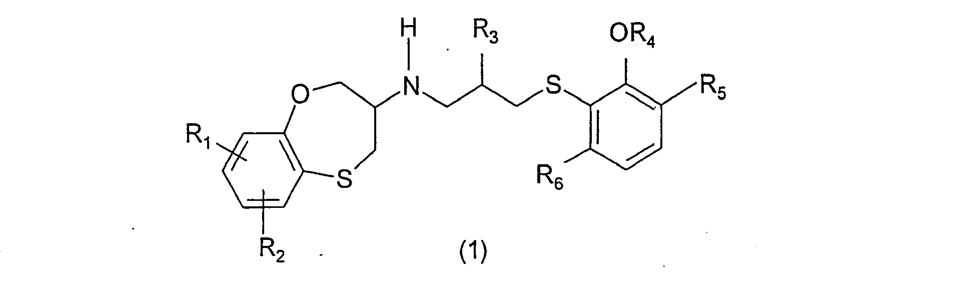 dans laquelle :
R₁ et R₂, identiques ou différents, représentent :
- un atome d'hydrogène ;
- un atome de fluor ou un atome de chlore ;
- un groupe hydroxy ;
- un radical alkyle choisi parmi les radicaux méthyle, éthyle, propyle ou isopropyle ;
- un radical alcoxy choisi parmi les radicaux méthoxy, éthyloxy, propyloxy ou isopropyloxy ;
- lorsque les groupes R₁ et R₂ occupent des positions adjacentes sur le cycle aromatique, alors R₁R₂ représentent -CH₂CH₂CH₂-, -OCH₂CH₂-, -OCH₂O- ou - CH₂CH₂O- ;
R₃ représente :
- un radical alkyle, choisi parmi les radicaux méthyle, éthyle, propyle ou isopropyle ;
- un groupe hydroxy ou un radical méthoxy ;
R₄ représente :
- un atome d'hydrogène ou un radical méthyle ;
R₅ et R₆ identiques ou différents représentent :
- un atome d'hydrogène ;
- un radical alkyle choisi parmi les radicaux méthyle, éthyle ou isopropyle ;
- un radical alcoxy choisi parmi les radicaux méthoxy, éthyloxy ou isopropyloxy ;
- un radical alkylthio choisi parmi les radicaux méthylthio, éthylthio ou isopropylthio ;
- un radical alkylamino choisi parmi les radicaux N-méthylamino ou N,N-diméthylamino ;
ou R₄R₅ représentent un radical choisi parmi les radicaux -CH₂CH₂-, -CH₂O-, CH₂CH₂O-, -CH₂CH₂S-, -CH₂CH₂NR₄- et R₆ est tel que défini précédemment
**caractérisé en ce qu'**il met en oeuvre la réduction d'un amide de formule (9) 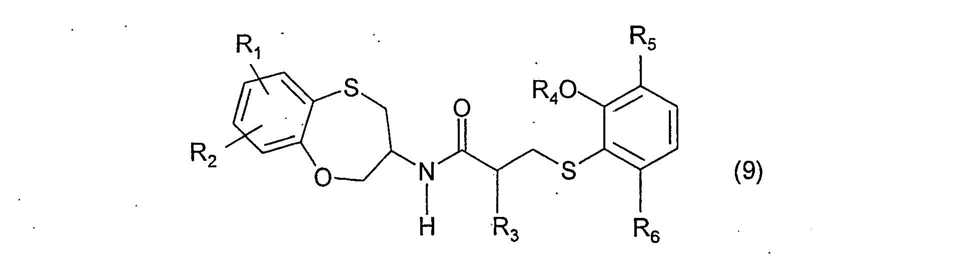 de préférence obtenu par condensation des intermédiaires de formule (5) et (7) ou (8) 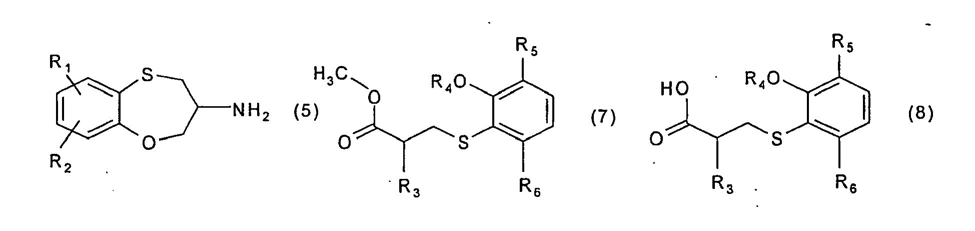 dans lesquelles les radicaux R₁, R₂, R₃, R₄, R₅ et R₆ ont les significations données ci-dessus à propos de la formule (1).

2. Procédé selon la revendication 1, **caractérisé en ce que** les 3,4-dihydro-2H-1,5-benzoxathiépin-3-amines de formule (5) 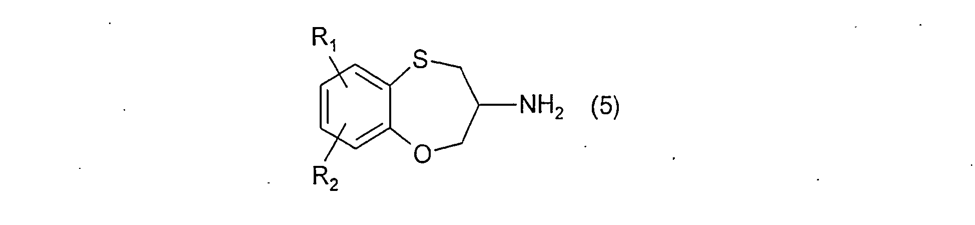 dans laquelle R₁ et R₂ ont les significations données à la revendication 1 à propos de la formule (1)
sont préparés par la mise en oeuvre des réactions suivantes :
- transformation du sel de diazonium de formule (2) en alkyl S-(2-fluoroaryl)-cystéinate de formule (3) au moyen d'un ester de cystine 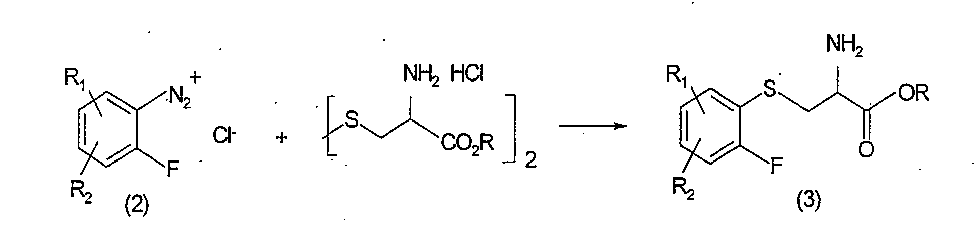
dans laquelle R représente un radical méthyle,
- réduction de ce composé par un agent donneur d'hydrure pour obtenir le 2-amino-3-[(2-fluoroaryl)sulfanyl]propan-1-ol de formule (4) 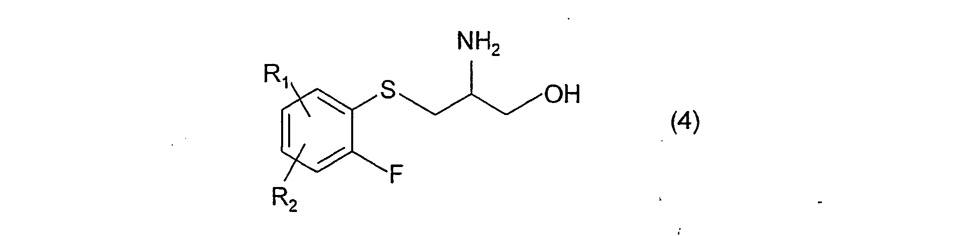
- cyclisation chimiosélective de ce composé pour donner les 3,4-dihydro-2H-1,5-benzoxathiépin-3-amines de formule (5) dans laquelle les radicaux R₁ et R₂ ont les significations données à propos de la formule (1).

3. Procédé selon la revendication 1, **caractérisé en ce que** les intermédiaires de formule (7) 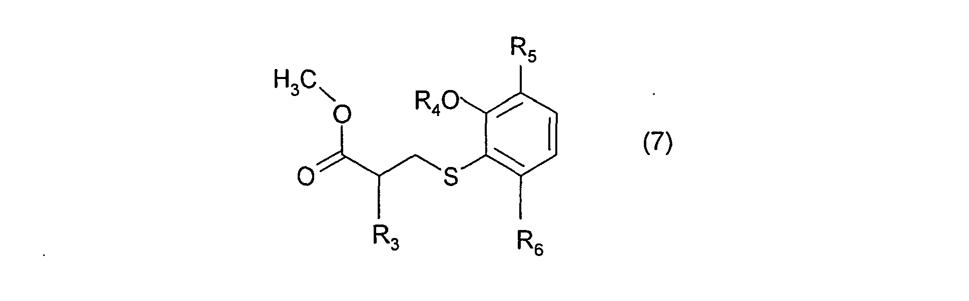 sont préparés par réaction d'un dérivé du type méthyl 3-[(méthylsulfonyl)oxy]propanoate substitué en position 2, de formule (6), avec un arylthiophénol approprié pour donner le composé de formule (7) 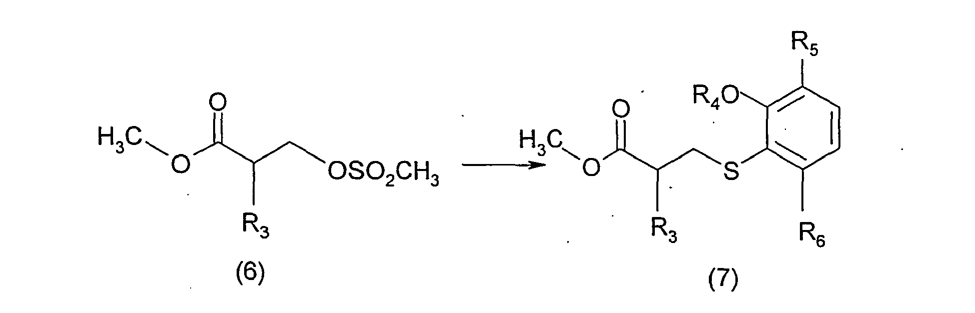 dans laquelle les radicaux R₃, R₄, R₅ et R₆ ont les significations données à propos de la formule (1).

4. Procédé selon la revendication 1, **caractérisé en ce que** les intermédiaires de formule (8) 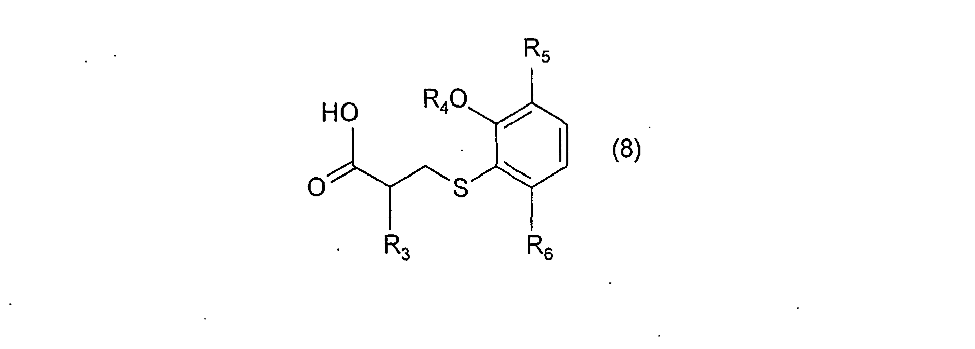 sont préparés par l'hydrolyse d'un composé de formule (7) en milieu basique pour donner l'acide correspondant de formule (8) 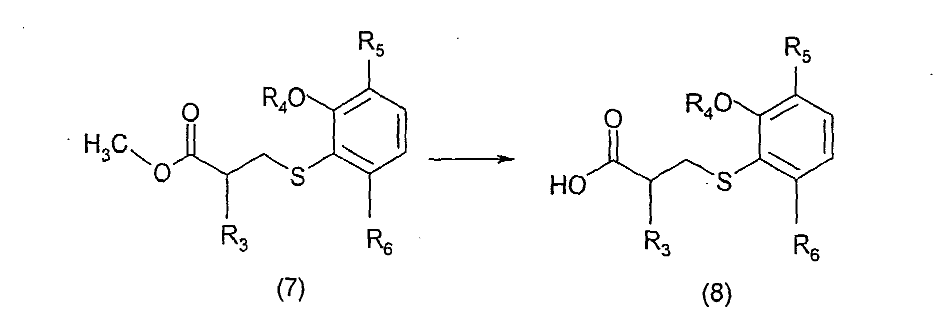 dans laquelle les radicaux R₃, R₄, R₅ et R₆ ont les significations données à propos de la formule (1).

5. Nouveaux intermédiaires de synthèse de formule générale (9) utilisés pour la préparation des composés de formule générale (1) selon le procédé de là revendication 1 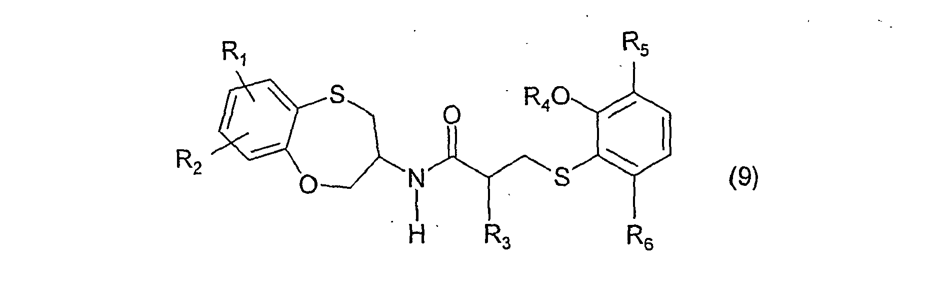 dans lesquelles les radicaux R₁, R₂, R₃, R₄, R₅ et R₆ ont les significations données à propos de la formule (1).

6. Nouveaux intermédiaires de synthèse de formule générale (7) utilisés pour la préparation des composés de formule générale (9) et (8) selon le procédé des revendications 1 ou 4, 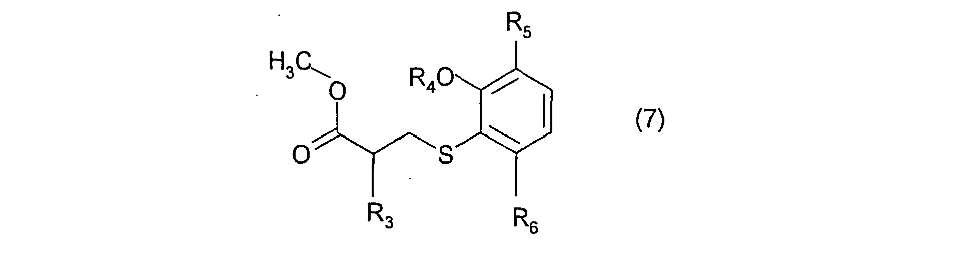 dans lesquelles les radicaux R₃, R₄, R₅ et R₆ ont les significations données à propos de la formule (1), R₃ étant différent d'un radical méthyle lorsque R₄, R₅ et R₆ représentent chacun un atome d'hydrogène.

7. Nouveaux intermédiaires de synthèse de formule générale (8) utilisés pour la préparation des composés de formule générale (9) selon le procédé de la revendication 1, 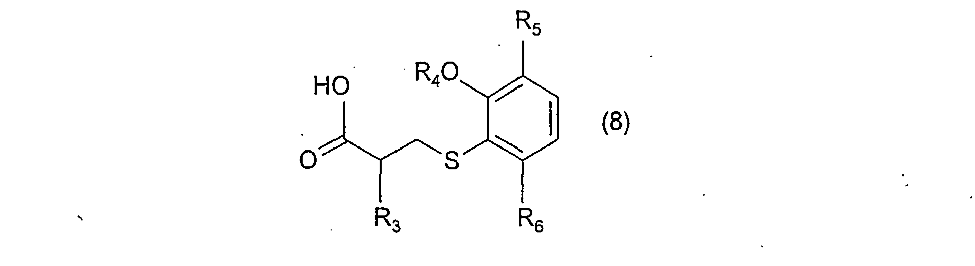 dans lesquelles les radicaux R₃, R₄, R₅ et R₆ ont les significations données à propos de la formule (1).

8. Procédé de préparation des composés de formule (1a), dérivés de ceux de la formule générale (1) 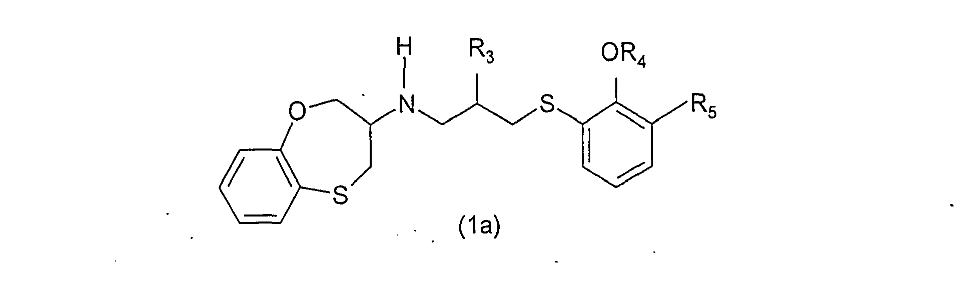 dans laquelle :
R₃ représente un radical alkyle choisi parmi les radicaux méthyle, éthyle ou isopropyle ;
R₄ représente un atome d'hydrogène ou un radical méthyle ;
R₅ représentent un atome d'hydrogène ou un radical méthyle ;
ou bien R₄R₅ représentent un radical -CH₂CH₂-
**caractérisé en ce qu'**il met en oeuvre la réduction d'un amide de formule (9a) 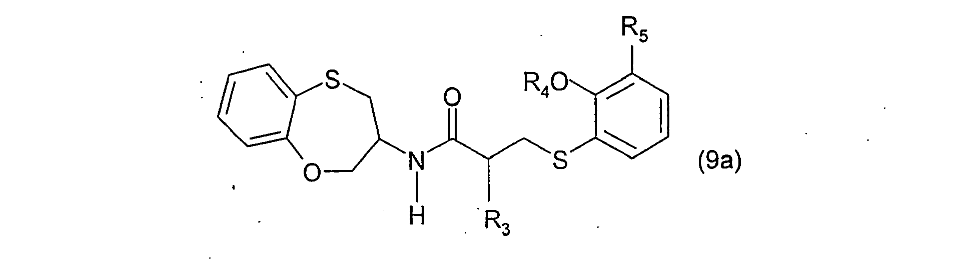 de préférence obtenu par condensation des intermédiaires de formule (5a) et (7a) ou (8a) 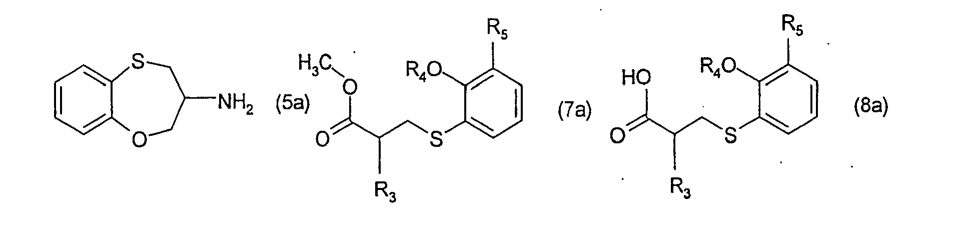 dans lesquelles les radicaux R₃, R₄ et R₅ ont les significations données ci-dessus à propos de la formule (1a).

9. Procédé selon la revendication 8, **caractérisé en ce que** les 3,4-dihydro-2H-1,5-benzoxathiépin-3-amines de formule (5a) 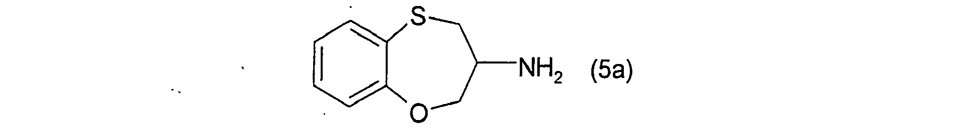 sont préparés par la mise en oeuvre des réactions suivantes :
- transformation du sel de diazonium de formule (2a) en alkyl S-(2-fluorophényl)-cystéinate de formule (3a) au moyen d'un ester de cystine 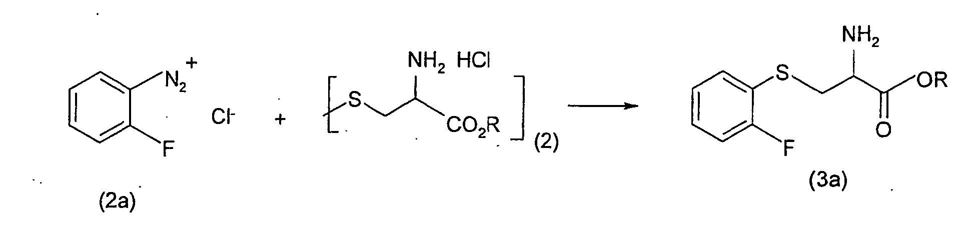
- réduction de ce composé par un agent donneur d'hydrure pour obtenir le 2-amino-3-[(2-fluorophényl)sulfanyl]propan-1-ol de formule (4a) 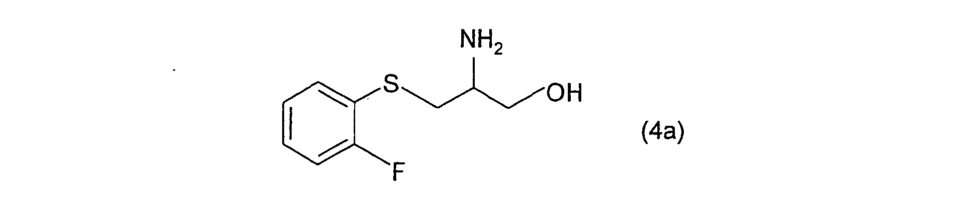
- cyclisation de ce composé en milieu basique pour donner les 3,4-dihydro-2H-1,5-benzoxathiépin-3-amines de formule (5a).

10. Procédé selon la revendication 8, **caractérisé en ce que** les intermédiaires de formule (7a) 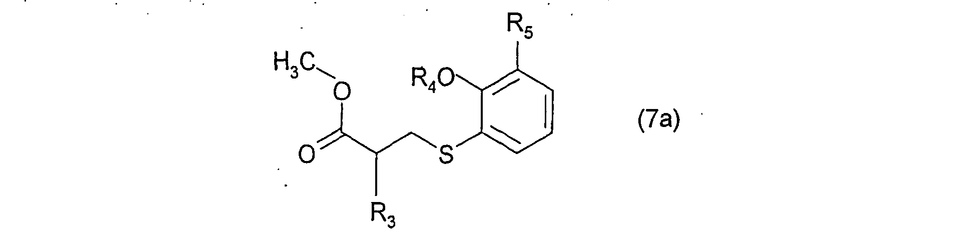 sont préparés par réaction d'un dérivé du type méthyl 3-[(méthylsulfonyl)oxy]propanoate substitué en position 2, de formule (6), avec un arylthiophénol approprié pour donner le composé de formule (7a) 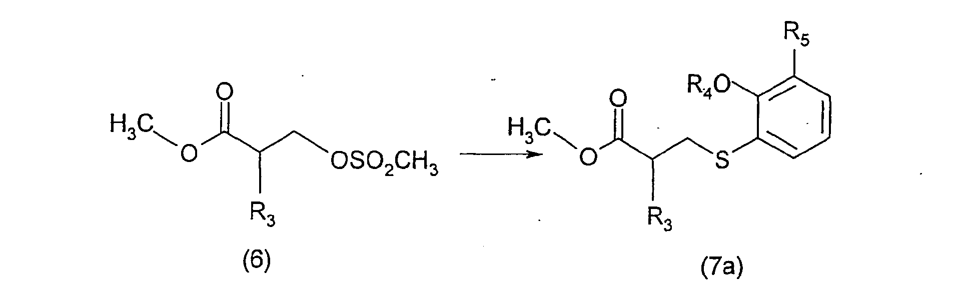 dans laquelle les radicaux R₃, R₄ et R₅ ont les significations données à propos de la formule (1a).

11. Procédé selon la revendication 8, **caractérisé en ce que** les intermédiaires de formule (8a) 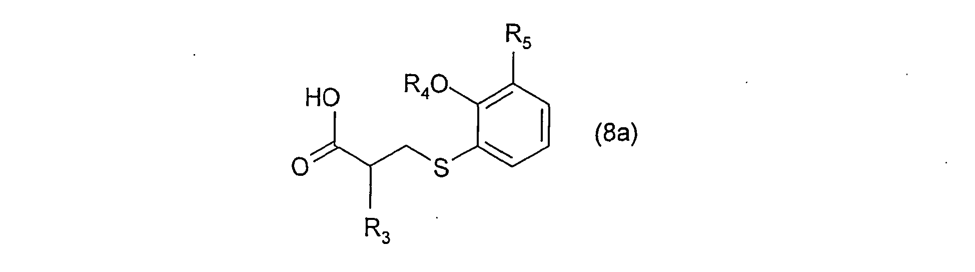 sont préparés par hydrolyse d'un composé de formule (7a) en milieu basique pour donner l'acide correspondant de formule (8a) 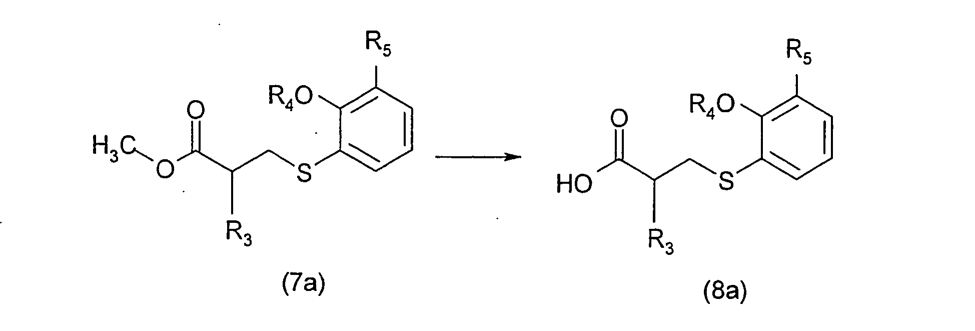 dans laquelle les radicaux R₃, R₄ et R₅ ont les significations données à propos de la formule (1a).

12. Nouveaux intermédiaires de synthèse de formule (9a) utilisés pour la préparation des composés de formule (1a) selon le procédé de la revendication 8, 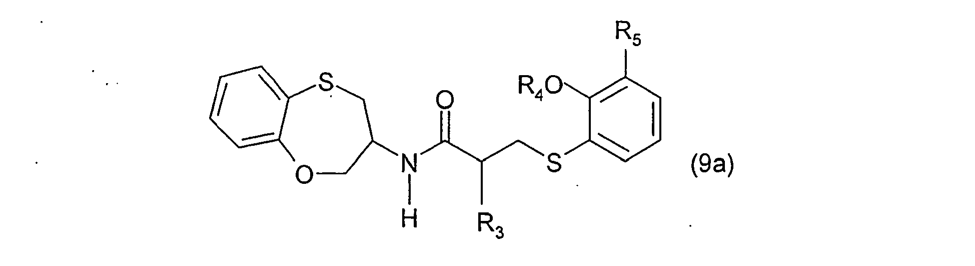 dans lesquelles les radicaux R₃, R₄ et R₅ ont les significations données à propos de la formule (1a).

13. Nouveaux intermédiaires de synthèse de formule (7a) utilisés pour la préparation des composés de formule générale (9a) et (8a) selon le procédé des revendications 8 ou 11, 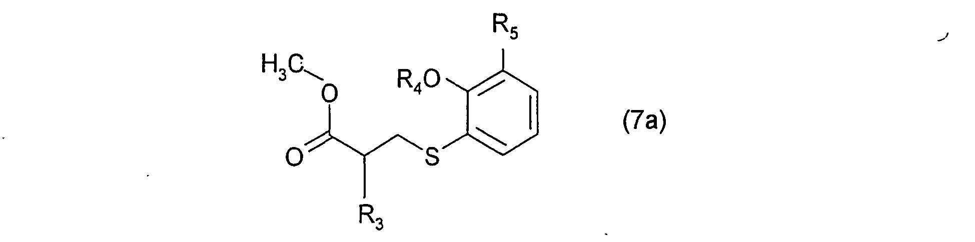 dans lesquelles les radicaux R₃, R₄ et R₅ ont les significations données à propos de la formule (1a), R₃ étant différent d'un radical méthyle lorsque R₄, et R₅ représentent chacun un atome d'hydrogène.

14. Nouveaux intermédiaires de synthèse de formule (8a) utilisés pour la préparation des composés de formule (9a) selon le procédé de la revendication 8, 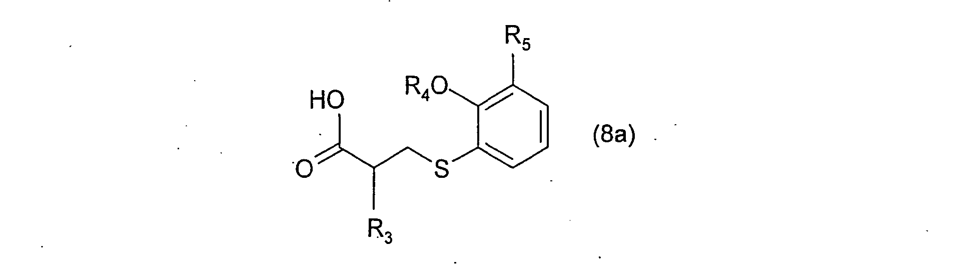 dans lesquelles les radicaux R₃, R₄ et R₅ ont les significations données à propos de la formule (1a).

15. Procédé de préparation des dérivés de benzoxathiépines de formules générales (1) et (1a), selon l'une des revendications 1 à 4 et 8 à 11, **caractérisé en ce que** l'on prépare des composés de formule (1) ou (1a) qui sont de configuration absolue (R) au niveau de l'atome de carbone stéréogénique C(3) du fragment 3,4-dihydro-2H-1,5-benzoxathiépine et de configuration absolue (S) au niveau de l'atome de carbone stéréogénique qui porte le groupe R₃.

16. Composé intermédiaire de formules (9) et (9a) selon les revendications 5 et 12 dans lesquels l'atome de carbone stéréogénique C(3) du fragment 3,4-dihydro-2H-1,5-benzoxathiépine est de configuration absolue (R) et l'atome de carbone stéréogénique qui porte le groupe R₃ est de configuration absolue (S).

17. Composé intermédiaire de formules (7) et (7a) selon les revendications 6 et 13 dans lesquels l'atome de carbone stéréogénique qui porte le groupe R₃ est de configuration absolue (S).

18. Composé intermédiaire de formules (8) et (8a) selon les revendications 7 et 14 dans lesquels l'atome de carbone stéréogénique qui porte le groupe R₃ est de configuration absolue (S).

19. Procédé selon l'une des revendications 8 à 11, **caractérisé en ce que** l'on prépare les composés suivants :
N-{3-[(2-Méthoxyphényl)sulfanyl]-2-méthylpropyl}-3,4-dihydro-2H-1,5-benzoxathiépin-3-amine ;
2-{[3-(3,4-Dihydro-2H-1,5-benzoxathiépin-3-ylamino)-2-méthylpropyl]sulfanyl}-6-méthylphénol,
(3R)-N-{(2S)-3-[(2-Méthoxyphényl)sulfanyl]-2-méthylpropyl}-3,4-dihydro-2H-1,5-benzoxathiépin-3-amine ;
2-({(2S)-3-[(3R)-3,4-Dihydro-2H-1,5-benzoxathiépin-3-ylamino]-2-méthylpropyl}sulfanyl)-6-méthylphénol,
leurs sels d'addition et les hydrates de ces sels d'addition avec les acides minéraux ou les acides organiques pharmaceutiquement acceptables.

## Claims

1. A method for preparing formula (1) benzoxathiepine derivatives 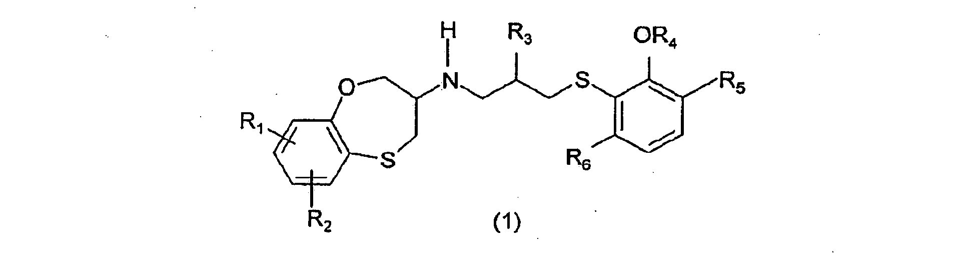 in which:
R₁ and R₂, identical or different, represent:
- a hydrogen atom;
- a fluorine atom or a chlorine atom;
- a hydroxy group;
- an alkyl radical, chosen from among the radicals methyl, ethyl, propyl, or isopropyl;
- an alkoxy radical, chosen from among the radicals methoxy, ethyloxy, propyloxy, or isopropyloxy;
- when groups R₁ and R₂ occupy adjacent positions on the aromatic ring, then R₁R₂ represents -CH₂CH₂CH₂-, -OCH₂CH₂-, -OCH₂O-, or -CH₂CH₂O-;
R₃ represents:
- an alkyl radical, chosen from among the radicals methyl, ethyl, propyl, or isopropyl;
- a hydroxy group or a methoxy radical;
R₄ represents:
- a hydrogen atom or a methyl radical;
R₅ and R₆, identical or different, represent:
- a hydrogen atom;
- an alkyl radical, chosen from among the radicals methyl, ethyl, or isopropyl;
- an alkoxy radical, chosen from among the radicals methoxy, ethyloxy, or isopropyloxy;
- an alkylthio radical, chosen from among radicals methylthio, ethylthio, or isopropylthio,
- an alkylamino radical, chosen from among the radicals N-methylamino or N,N-dimethylamino;
or R₄R₅ represents a radical chosen from among the radicals -CH₂CH₂-, -CH₂O-, -CH₂CH₂O- -CH₂CH₂S-, -CH₂CH₂NR₄- and R₆ is as defined previously, **characterized** such that it causes the reduction of an amide of formula (9) 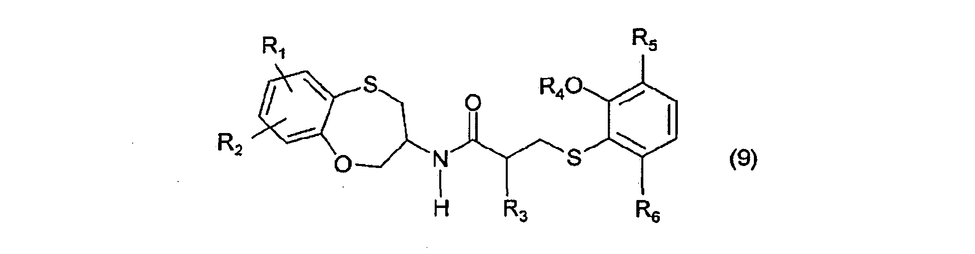 preferably obtained by condensation of the intermediates of formula (5), (7), or (8) 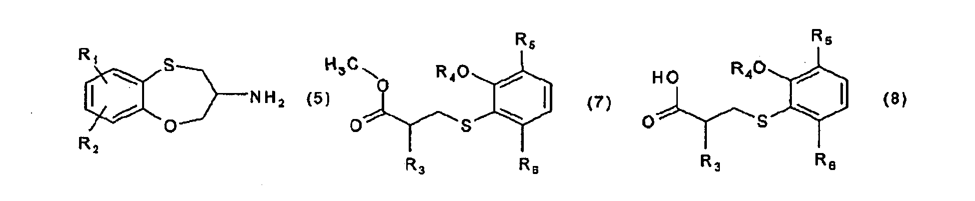 in which radicals R₁, R₂, R₃, R₄, R₅, and R₆ have the meaning given above with regard to formula (1).

2. A method according to claim 1, wherein the 3,4-dihydro-2H-1,5-benzoxathiepin-3-amines of formula (5) 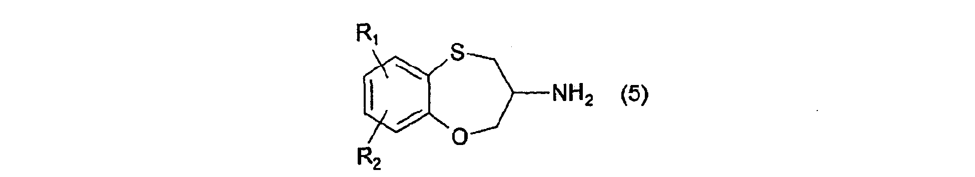 in which R₁ and R₂ have the meaning given in claim 1 with regard to formula (1)
are prepared by carrying out the following reactions:
- transformation of the diazonium salt of formula (2) into the alkyl S-(2-fluoroaryl)-cysteinate of formula (3) by means of a cystine ester 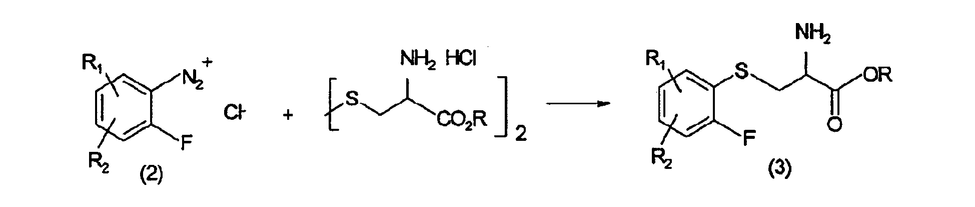
in which R represents a methyl radical,
- reduction of this compound by a hydride-donating agent to obtain the 2-amino-3-[(2-fluoroaryl)sulfanyl]propan-1-ol of formula (4) 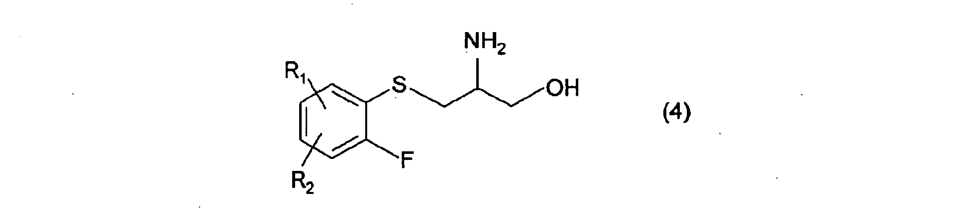
- chemoselective cyclization of this compound to yield the 3,4-dihydro-2H-1,5-benzoxathiepin-3-amines of formula (5) in which radicals R₁ and R₂ have the meaning given with regard to formula (1).

3. A method according to claim 1, wherein the intermediates of formula (7) 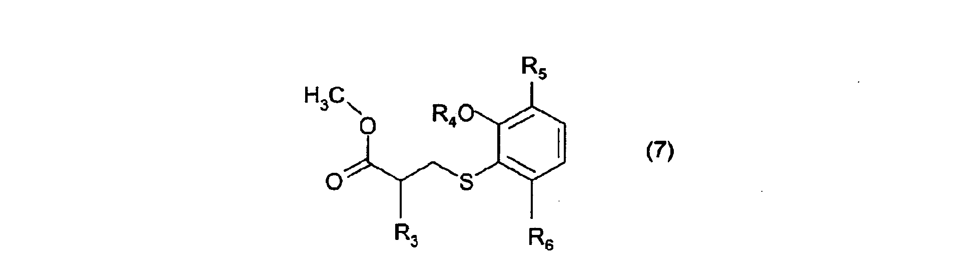 are prepared by the reaction of a formula (6) derivative of the type with 3-[(methylsulfonyl)oxy]propanoate substituted in position 2, with an appropriate arylthio phenol to yield the compound of formula (7) 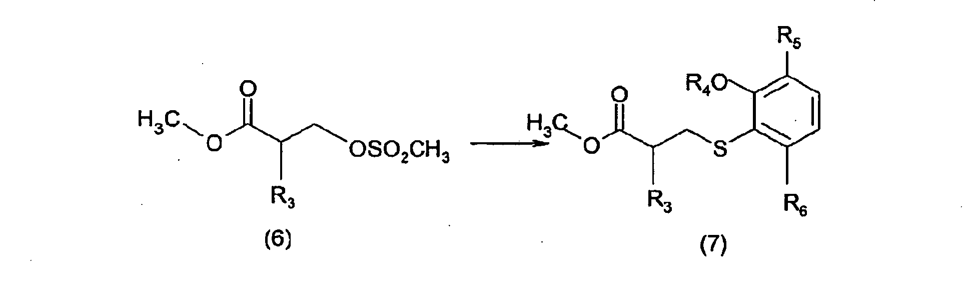 in which radicals R₃, R₄, R₅, and R₆ have the meaning given with regard to formula (1).

4. A method according to claim 1, wherein the intermediates of formula (8) 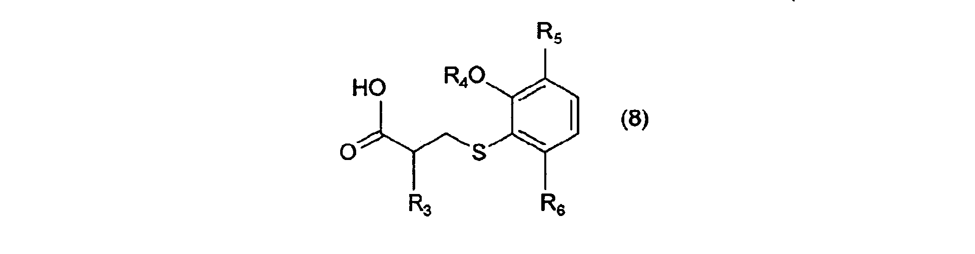 are prepared by the hydrolysis of a compound of formula (7) in a basic mixture to yield the acid corresponding to formula(8) 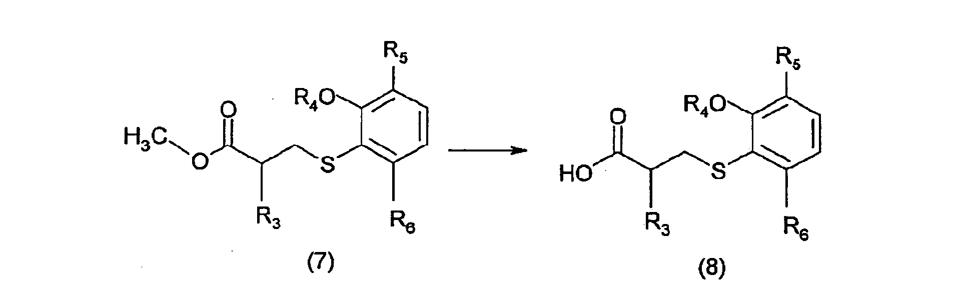 in which radicals R₃, R₄, R₅, and R₆ have the meaning given with regard to formula (1).

5. New synthetic intermediates of general formula (9) used for the preparation of compounds of general formula (1) according to the method of claim 1 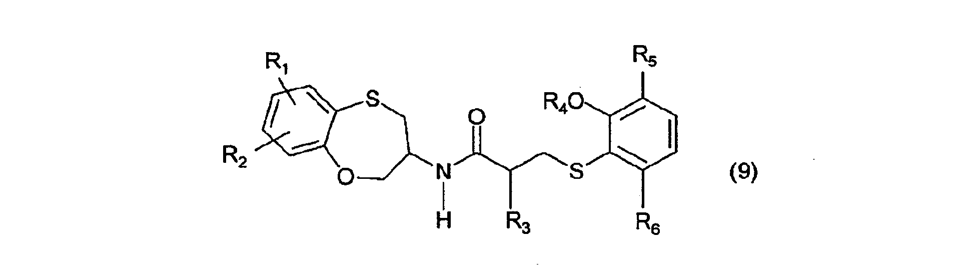 in which radicals R₁, R₂, R₃, R₄, R₅, and R₆ have the meaning given with regard to formula (1).

6. New synthetic intermediates of general formula (7) used for the preparation of compounds of general formulas (9) and (8) according to the methods of claims 1 or 4, 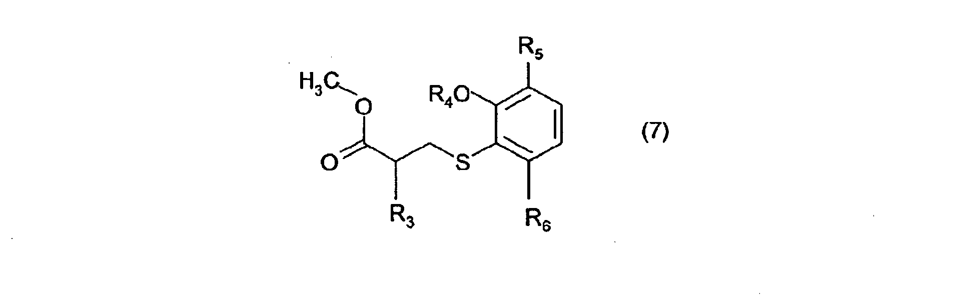 in which radicals R₃, R₄, R₅, and R₆ have the meaning given with regard to formula (1), R₃ being other than a methyl radical when R₄, R₅, and R₆ each represent a hydrogen atom.

7. New synthetic intermediates of general formula (8) used for the preparation of compounds of general formula (9) according to the method of claim 1, 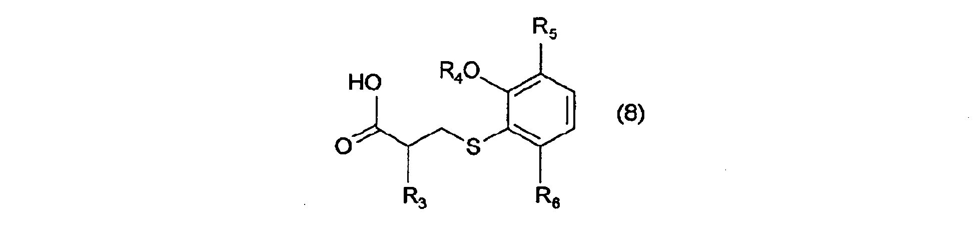 in which radicals R₃, R₄, R₅, and R₆ have the meaning given with regard to formula (1).

8. A method for preparing formula (la)compounds and derivatives of general formula (1) compounds 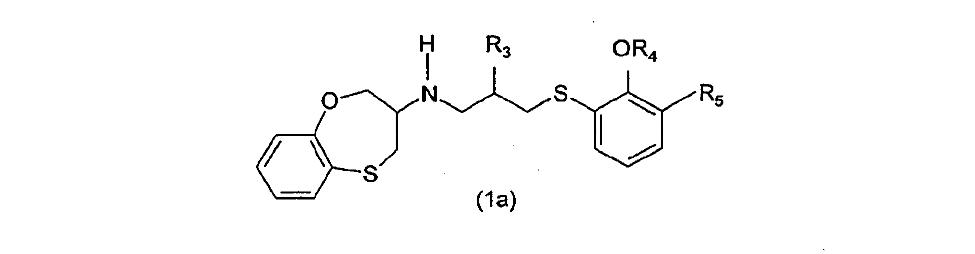 in which:
R₃ represents an alkyl radical chosen from among the radicals methyl, ethyl, or isopropyl;
R₄ represents a hydrogen atom or a methyl radical;
R₅ represents a hydrogen atom or a methyl radical;
or R₄R₅ represents a -CH₂CH₂- radical wherein it carries out the reduction of an amide of formula (9a) 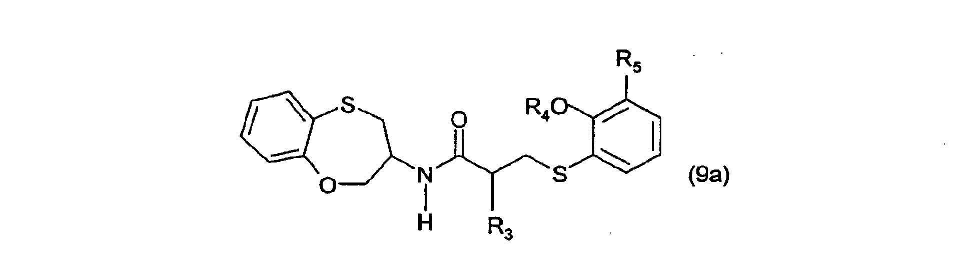 preferably obtained by condensation of the intermediates of formulas (5a), (7a), or (8a) 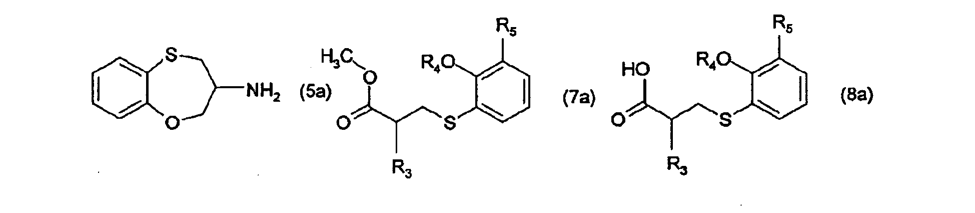 in which radicals R₃, R₄, and R₅ have the meaning given above with regard to formula (1a).

9. A method according to claim 8, wherein the 3,4-dihydro-2H-1,5-benzoxathiepin-3-amines of formula (5a) 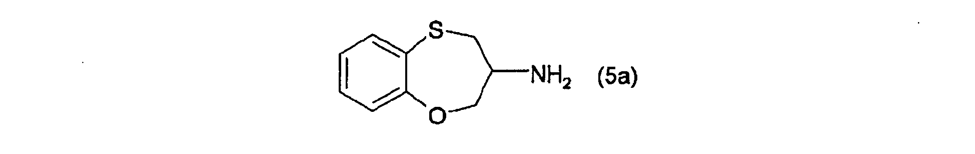 are prepared by carrying out the following reactions:
- transformation of the formula (2) diazonium salt into a S-(2-fluorophenyl)-cysteinate alkyl of formula (3a) by means of a cystine ester 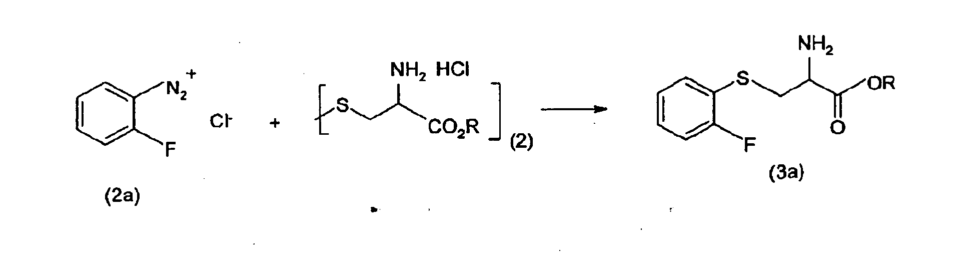
- reduction of this compound by a hydride-donating agent to obtain the 2-amino-3-[(2-fluorophenyl)sulfanyl]propan-1-ol of formula (4a) 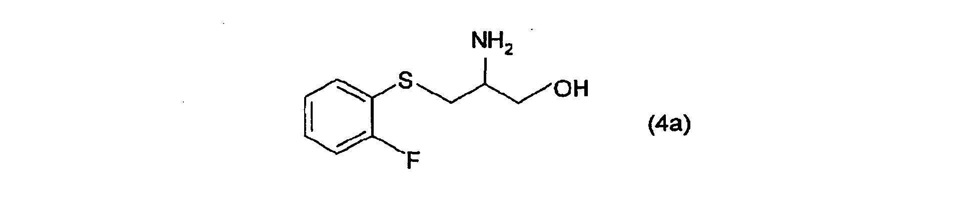
- cyclization of this compound in a basic mixture to yield the 3,4-dihydro-2H-1,5-benzoxathiepin-3-amines of formula (5a).

10. A method according to claim 8, wherein the intermediates of formula (7a) 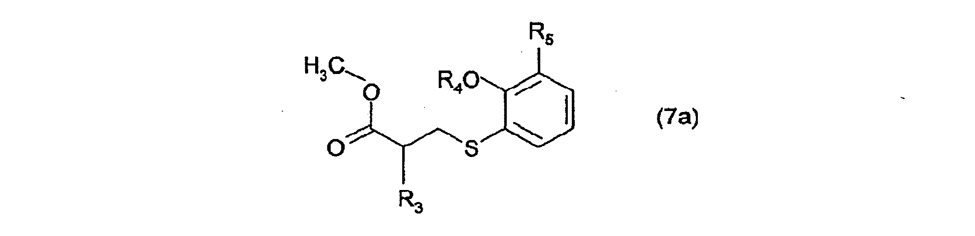 are prepared by the reaction of a formula (6) derivative of the type with 3-[(methylsulfonyl)oxy]propanoate substituted in position 2, with an appropriate arylthio phenol to yield the compound of formula (7a) 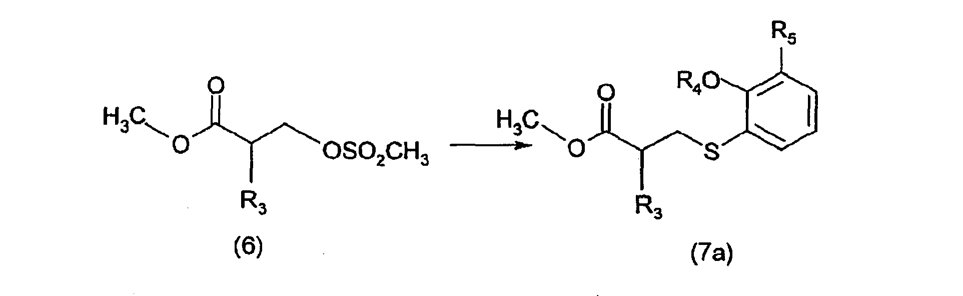 in which radicals R₃, R₄, and R₅ have the meaning given with regard to formula (1a).

11. A method according to claim 8, wherein the intermediates of formula (8a) 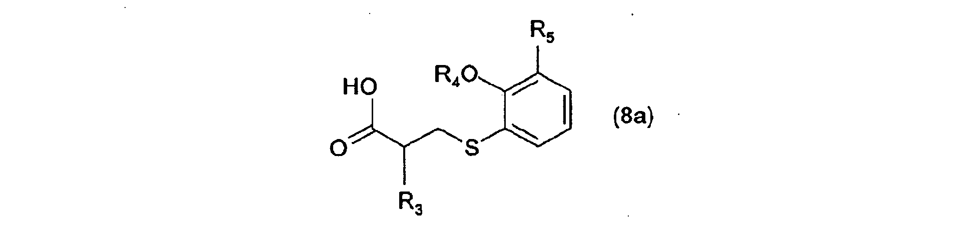 are prepared by the hydrolysis of a compound of formula (7a) in a basic mixture to yield the acid corresponding to formula (8a) 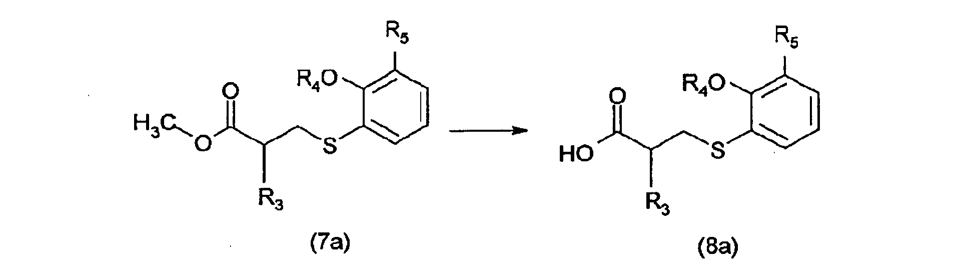 in which radicals R₃, R₄, and R₅ have the meaning given with regard to formula (1a).

12. New synthetic intermediates of general formula (9a) used for the preparation of compounds of general formula (1a) according to the method of claim 8, 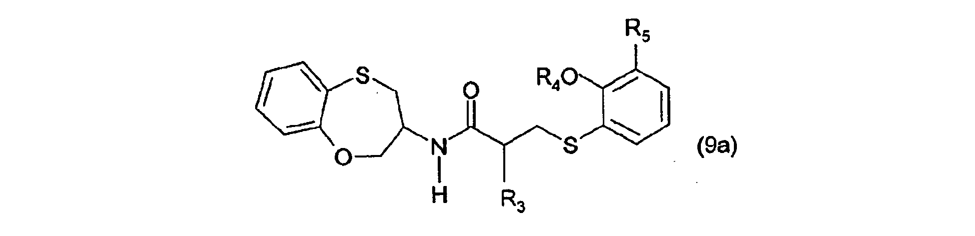 in which radicals R₃, R₄, and R₅ have the meaning given with regard to formula (1a).

13. New synthetic intermediates of general formula (7a) used for the preparation of compounds of general formulas (9a) and (8a) according to the methods of claims 8 or 11, 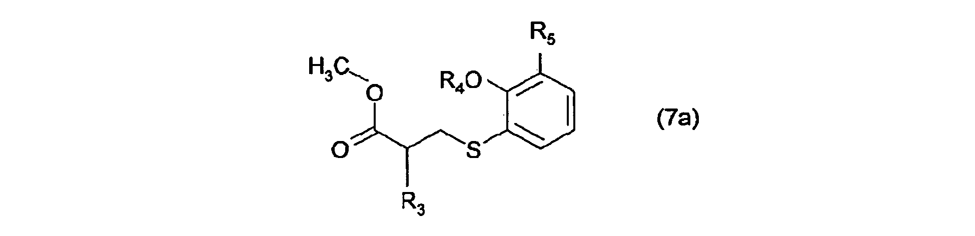 in which radicals R₃, R₄, and R₅ have the meaning given with regard to formula (1a), R₃ being other than a methyl radical when R₄ and R₅ each represent a hydrogen atom.

14. New synthetic intermediates of general formula (8a) used for the preparation of compounds of general formula (9a) according to the method of claim 8, 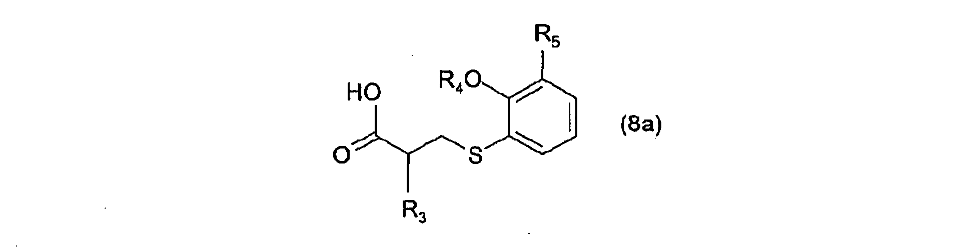 in which radicals R₃, R₄, and R₅ have the meaning given with regard to formula (1a).

15. A method for preparing derivatives of benzoxathiepines of general formulas (1) and (1a), according to ,one of the claims 1 to 4 and 8 to 11, wherein the compounds of formula (1) or (1a), in which the stereogenic C(3) carbon atom of fragment 3,4-dihydro-2H-1,5-benzoxathiepine is of absolute configuration (R) and the stereogenic carbon atom that carries the R₃ group is of absolute configuration (S), are prepared.

16. Intermediate compounds of formulas (9) and (9a) according to claims 5 and 12 in which the stereogenic C(3) carbon atom of fragment 3,4-dihydro-2H-1,5-benzoxathiepine is of absolute configuration (R) and the stereogenic carbon atom that carries the R₃ group is of absolute configuration (S).

17. Intermediate compounds of formulas (7) and (7a) according to claims 6 and 13 in which the stereogenic carbon atom that carries the R₃ group is of absolute configuration (S).

18. Intermediate compounds of formulas (8) and (8a) according to claims 7 and 14 in which the stereogenic carbon atom that carries the R₃ group is of absolute configuration (S).

19. Method according to one of the claims 8 to 11, wherein the following compounds are prepared:
N-{3-[(2-Methoxyphenyl)sulfanyl]-2-methylpropyl}-3,4-dihydro-2H-1,5-benzoxathiepin-3-amine;
2-{[3-(3,4-Dihydro-2H-1,5-benzoxathiepin-3-ylamino)-2-methylpropyl]sulfanyl}-6-methylphenol,
(3R)-N-{(2S)-3-[(2-Methoxyphenyl)sulfanyl]-2-methylpropyl}-3,4-dihydro-2H-1,5-benzoxathiepin-3-amine;
2-({(2S)-3-[(3R)-3,4-Dihydro-2H-1,5-benzoxathiepin-3-ylamino]-2-methylpropyl}sulfanyl)-6-methylphenol,
their addition salts and the hydrates of these addition salts with mineral acids or pharmaceutically acceptable organic acids.

## Patentansprüche

1. Verfahren zur Herstellung von Benzoxathiepin-Derivaten der Formel (1) 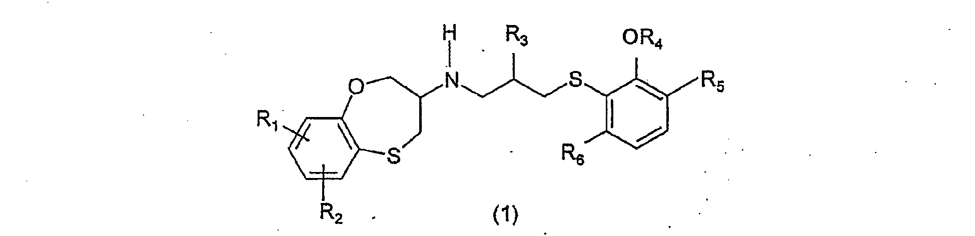 in der:
R₁ und R₂, identisch oder verschieden, darstellen:
- ein Wasserstoffatom;
- ein Fluoratom oder ein Chloratom;
- eine Hydroxygruppe;
- einen Alkylrest, der aus dem Methyl-, Ethyl-, Propyl- oder Isopropylrest ausgewählt ist;
- einen Alkoxyrest, der aus dem Methoxy-, Ethyloxy-, Propyloxy- oder Isopropyloxyrest ausgewählt ist;
- wenn die Gruppen R₁ und R₂ benachbarte Positionen am aromatischen Cyclus einnehmen, R₁R₂ -CH₂CH₂CH₂-, -OCH₂CH₂-, -OCH₂O- oder -CH₂CH₂O- darstellen;
R₃ darstellt:
- einen Alkylrest, der aus dem Methyl-, Ethyl-, Propyl- oder Isopropylrest ausgewählt ist;
- eine Hydroxygruppe oder einen Methoxyrest;
R₄ darstellt:
- ein Wasserstoffatom oder einen Methylrest;
R₅ und R₆, identisch oder verschieden, darstellen:
- ein Wasserstoffatom;
- einen Alkylrest, der aus dem Methyl-, Ethyl- oder Isopropylrest ausgewählt ist;
- einen Alkoxyrest, der aus dem Methoxy-, Ethyloxy- oder Isopropyloxyrest ausgewählt ist;
- einen Alkylthiorest, der aus dem Methylthio-, Ethylthio- oder Isopropylthiorest ausgewählt ist;
- einen Alkylaminorest, der aus dem N-Methylamino- oder N,N-Dimethylaminorest ausgewählt ist;
- oder R₄R₅ einen Rest darstellen, der aus den Resten -CH₂CH₂-, -CH₂O-, -CH₂CH₂O-, -CH₂CH₂S-, -CH₂CH₂NR₄- ausgewählt ist und R₆ wie vorstehend definiert ist,
**dadurch gekennzeichnet, dass** es die Reduktion eines Amids der Formel (9) 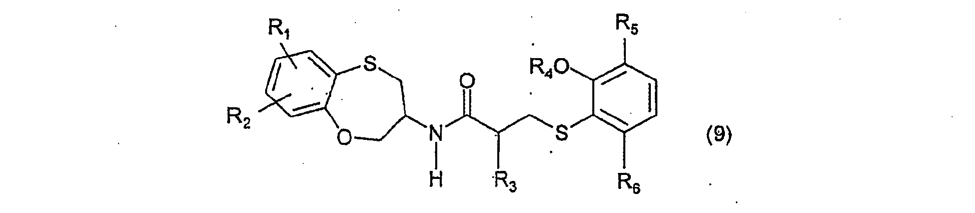 verwendet, das vorzugsweise durch Kondensation der Zwischenprodukte der Formel (5) und (7) oder (8) 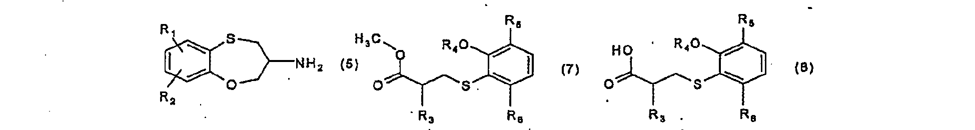 erhalten wurde, in denen die Reste R₁, R₂, R₃, R₄, R₅ und R₆ die vorstehend mit Bezug auf die Formel (1) gegebenen Bedeutungen aufweisen.

2. Verfahren nach Anspruch 1, **dadurch gekennzeichnet, dass** die 3,4-Dihydro-2H-1,5-benzoxathiepin-3-amine der Formel (5) 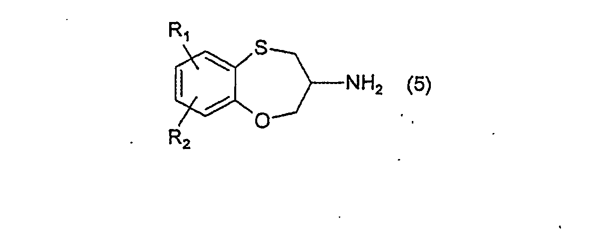 in der R₁ und R₂ die in Anspruch 1 mit Bezug auf die Formel (1) angegebenen Bedeutungen aufweisen,
hergestellt werden, indem man die folgenden Reaktionen durchführt:
- Überführung des Diazoniumsalzes der Formel (2) in ein Alkyl-S-(2-fluoraryl)cysteinat der Formel (3) mittels eines Cystinesters 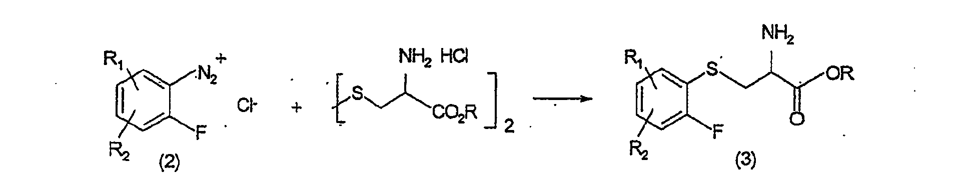 in der R einen Methylrest darstellt,
- Reduktion dieser Verbindung durch ein Hydrid-Donor-Mittel, um das 2-Amino-3-[(2-fluoraryl)sulfanyl]propan-1-ol der Formel (4) 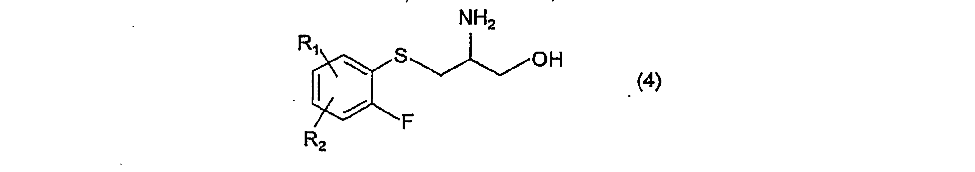 zu erhalten,
- chemoselektive Cyclisierung dieser Verbindung, um die 3,4-Dihydro-2H-1,5-benzoxathiepin-3-amine der Formel (5) zu ergeben, in der die Reste R₁ und R₂ die mit Bezug auf die Formel (1) gegebenen Bedeutungen aufweisen.

3. Verfahren nach Anspruch 1, **dadurch gekennzeichnet, dass** die Zwischenprodukte der Formel (7) 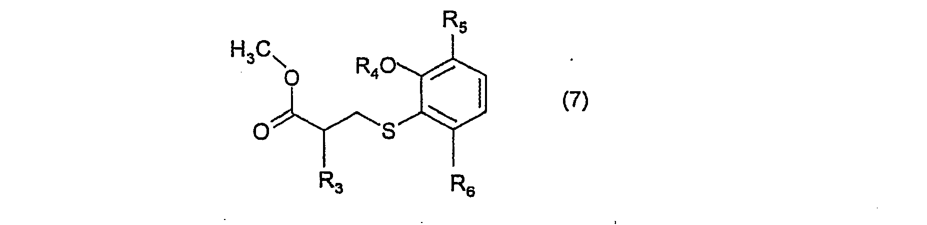 durch Umsetzung eines Derivats vom Typ Methyl-3-[(methylsulfonyl)-oxy]propanoat, das an Position 2 substituiert ist, der Formel (6) mit einem geeigneten Arylthiophenol hergestellt werden, um die Verbindung der Formel (7) zu ergeben 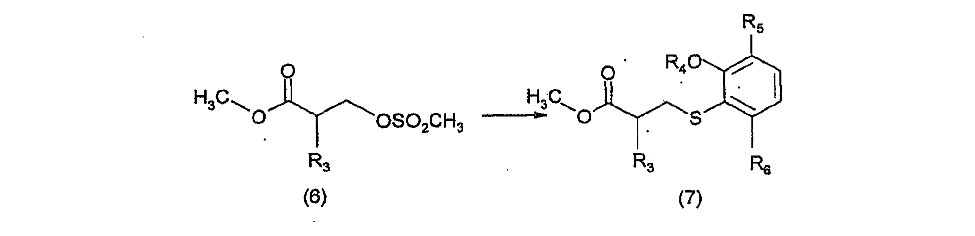 in der die Reste R₃, R₄, R₅ und R₆ die mit Bezug auf die Formel (1) angegebenen Bedeutungen aufweisen.

4. Verfahren nach Anspruch 1, **dadurch gekennzeichnet, dass** die Zwischenprodukte der Formel (8) 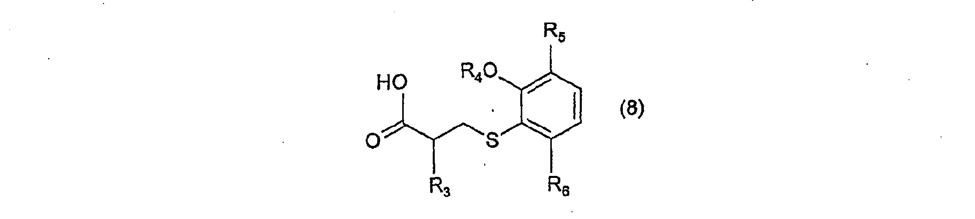 durch Hydrolyse einer Verbindung der Formel (7) in basischem Medium hergestellt werden, um die entsprechende Säure der Formel (8) zu ergeben 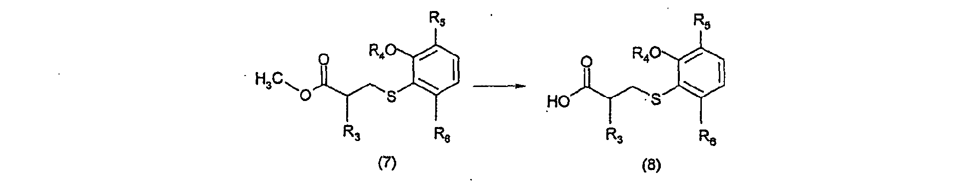 in der die Reste R₃, R₄, R₅ und R₆ die mit Bezug auf die Formel (1) angegebenen Bedeutungen aufweisen.

5. Neue Synthesezwischenprodukte der allgemeinen Formel (9), die zur Herstellung von Verbindungen der allgemeinen Formel (1) gemäß dem Verfahren nach Anspruch 1 verwendet werden 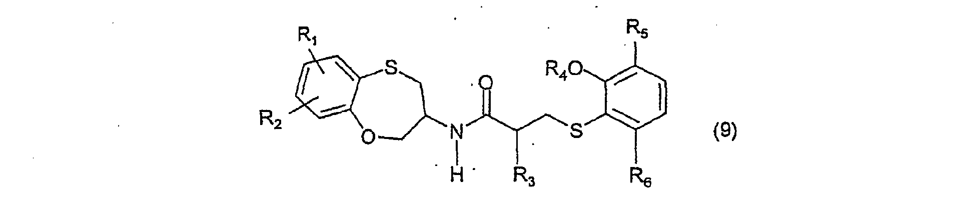 in denen die Reste R₁, R₂, R₃, R₄, R₅ und R₆ die mit Bezug auf die Formel (1) angegebenen Bedeutungen aufweisen.

6. Neue Synthesezwischenprodukte der allgemeinen Formel (7), die für die Herstellung von Verbindungen der allgemeinen Formel (9) und (8) gemäß dem Verfahren der Ansprüche 1 oder 4 verwendet werden 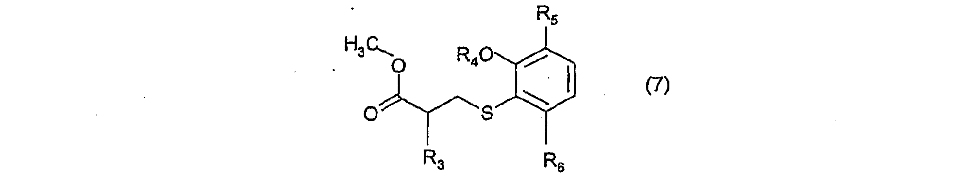 in denen die Reste R₃, R₄, R₅ und R₆ die mit Bezug auf die Formel (1) angegebenen Bedeutungen aufweisen, wobei R₃ von einem Methylrest verschieden ist, wenn R₄, R₅ und R₆ jeweils ein Wasserstoffatom darstellen.

7. Neue Synthesezwischenprodukte der allgemeinen Formel (8), die für die Herstellung von Verbindungen der allgemeinen Formel (9) gemäß dem Verfahren von Anspruch 1 verwendet werden, 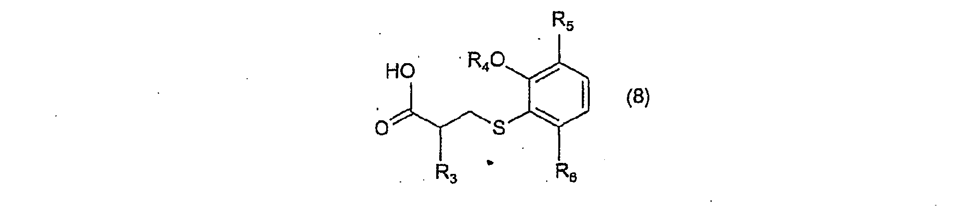 in denen die Reste R₃, R₄, R₅ und R₆ die mit Bezug auf die Formel (1) angegebenen Bedeutungen aufweisen.

8. Verfahren zur Herstellung von Verbindungen der Formel (1a), die Derivate jener der allgemeinen Formel (1) sind 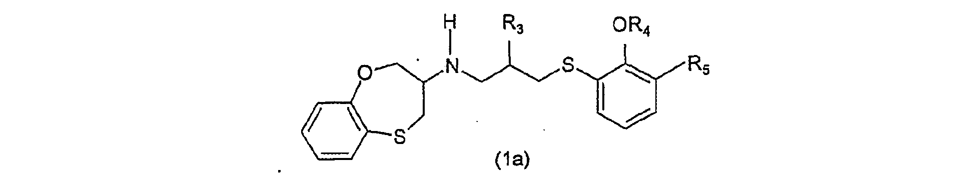 in der:
R₃ einen Alkylrest darstellt, der aus dem Methyl-, Ethyl- oder Isopropylrest ausgewählt ist;
R₄ ein Wasserstoffatom oder einen Methylrest darstellt;
R₅ ein Wasserstoffatom oder einen Methylrest darstellt;
oder auch R₄R₅ einen Rest -CH₂CH₂- darstellen,
**dadurch gekennzeichnet, dass** es die Reduktion eines Amids der Formel (9a) verwendet, 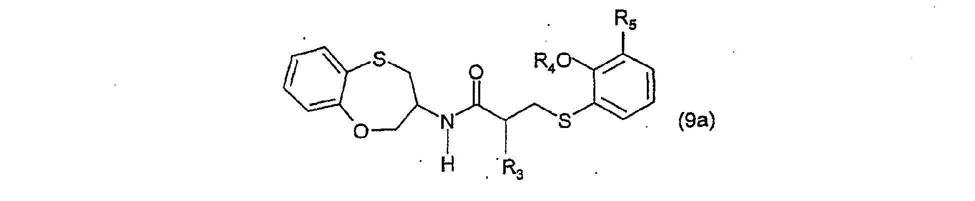 das bevorzugt durch Kondensation der Zwischenprodukte der Formel (5a) und (7a) oder (8a) erhalten wird 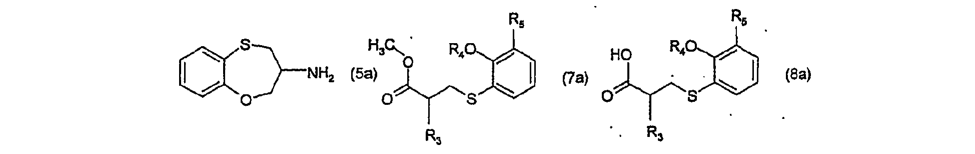 in denen die Reste R₃, R₄ und R₅ die vorstehend mit Bezug auf die Formel (1a) angegebenen Bedeutungen aufweisen.

9. Verfahren nach Anspruch 8, **dadurch gekennzeichnet, dass** die 3,4-Dihydro-2H-1,5-benzoxathiepin-3-amine der Formel (5a) 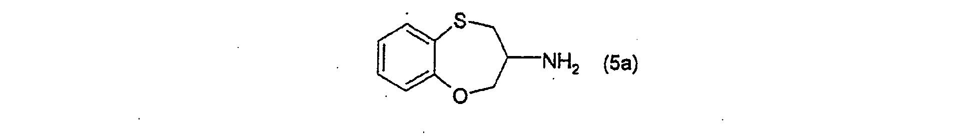 durch den Einsatz der folgenden Reaktionen hergestellt werden:
- Überführung des Diazoniumsalzes der Formel (2a) in das Alkyl-S-(2-fluorphenyl)cysteinat der Formel (3a) mittels eines Cystinesters, 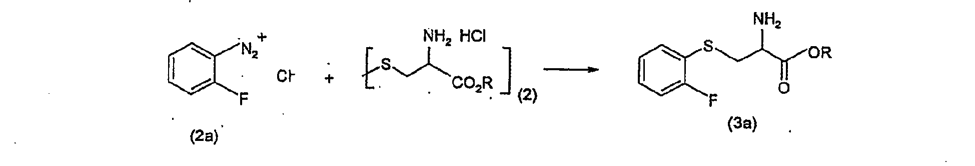
- Reduktion dieser Verbindung durch ein Hydrid-Donor-Mittel, um das 2-Amino-3-[(2-fluorphenyl)sulfanyl]propan-1-ol der Formel (4a) zu erhalten, 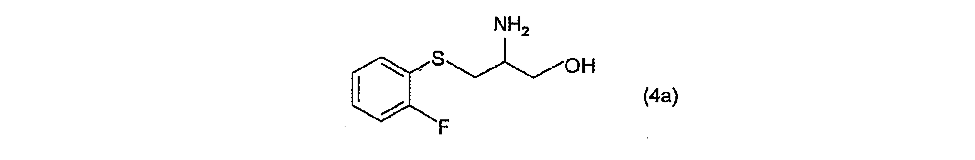
- Cyclisierung dieser Verbindung in basischem Medium, um die 3,4-Dihydro-2H-1,5-benzoxathiepin-3-amine der Formel (5a) zu ergeben.

10. Verfahren nach Anspruch 8, **dadurch gekennzeichnet, dass** die Zwischenprodukte der Formel (7a) 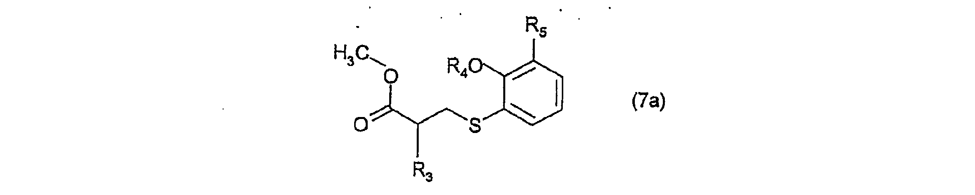 durch Umsetzung eines Derivats vom Typ Methyl-3-[(methylsulfonyl)-oxy]propanoat, das in Position 2 substituiert ist, der Formel (6) mit einem geeigneten Arylthiophenol hergestellt werden, um die Verbindung der Formel (7a) zu ergeben, 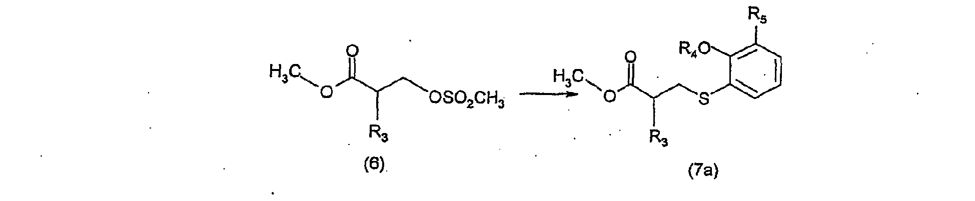 in der die Reste R₃, R₄ und R₅ die mit Bezug auf die Formel (1a) angegebenen Bedeutungen aufweisen.

11. Verfahren nach Anspruch 8, **dadurch gekennzeichnet, dass** die Zwischenprodukte der Formel (8a) 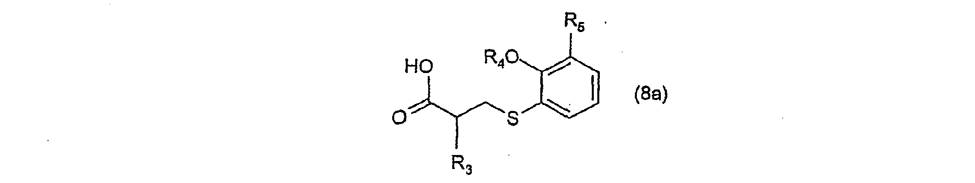 durch Hydrolyse einer Verbindung der Formel (7a) in basischem Medium hergestellt werden, um die entsprechende Säure der Formel (8a) zu ergeben, 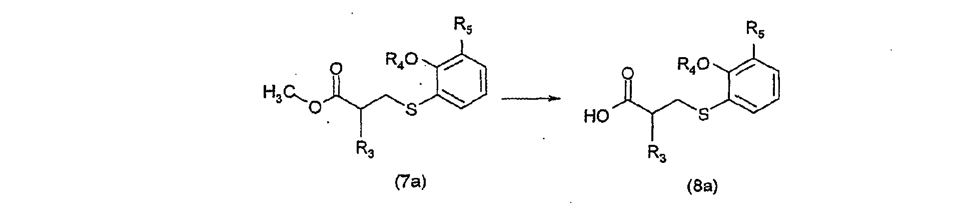 in der die Reste R₃, R₄ und R₅ die mit Bezug auf die Formel (1a) angegebenen Bedeutungen aufweisen.

12. Neue Synthesezwischenprodukte der Formel (9a), die für die Herstellung von Verbindungen der Formel (1a) gemäß dem Verfahren nach Anspruch 8 verwendet werden, 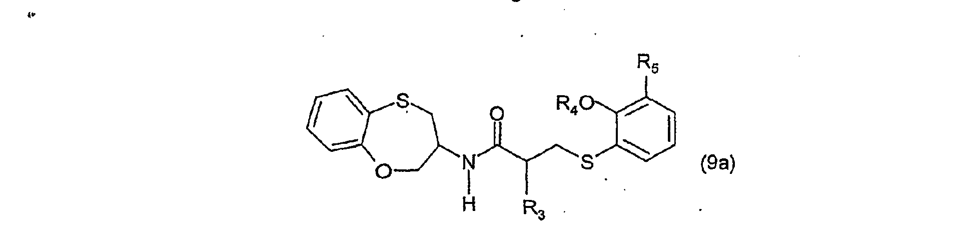 in denen die Reste R₃, R₄ und R₅ die mit Bezug auf die Formel (1a) angegebenen Bedeutungen aufweisen.

13. Neue Synthesezwischenprodukte der Formel (7a), die für die Herstellung von Verbindungen der allgemeinen Formel (9a) und (8a) gemäß dem Verfahren der Ansprüche 8 oder 11 verwendet werden, 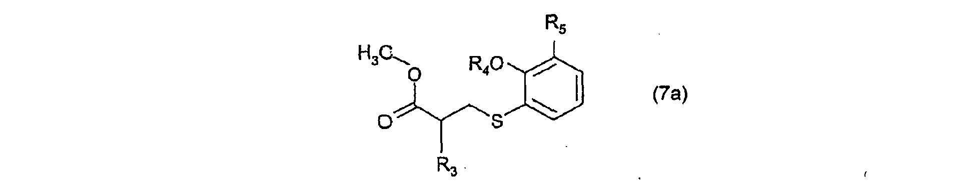 in denen die Reste R₃, R₄ und R₅ die mit Bezug auf die Formel (1a) angegebenen Bedeutungen aufweisen, wobei R₃ von einem Methylrest verschieden ist, wenn R₄ und R₅ jeweils ein Wasserstoffatom darstellen.

14. Neue Synthesezwischenprodukte der Formel (8a), die für die Herstellung von Verbindungen der Formel (9a) gemäß dem Verfahren nach Anspruch 8 verwendet werden, 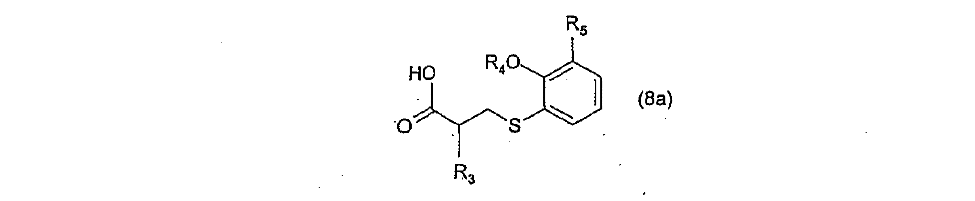 in denen die Reste R₃, R₄ und R₅ die mit Bezug auf die Formel (1a) angegebenen Bedeutungen aufweisen.

15. Verfahren zur Herstellung von Benzoxathiepin-Derivaten der allgemeinen Formeln (1) und (1a) gemäß einem der Ansprüche 1 bis 4 und 8 bis 11, **dadurch gekennzeichnet, dass** man Verbindungen der Formel (1) oder (1a) herstellt, welche die absolute Konfiguration (R) am stereogenen Kohlenstoffatom C(3) des Fragments 3,4-Dihydro-2H-1,5-benzoxathiepin und die absolute Konfiguration (S) am stereogenen Kohlenstoffatom, das die Gruppe R₃ trägt, aufweisen.

16. Zwischenprodukt-Verbindungen der Formeln (9), und (9a) gemäß den Ansprüchen 5 und 12, in denen das stereogene Kohlenstoffatom C(3) des Fragments 3,4-Dihydro-2H-1,5-benzoxathiepin die absolute Konfiguration (R) aufweist und das stereogene Kohlenstoffatom, das die Gruppe R₃ trägt, die absolute Konfiguration (S) aufweist.

17. Zwischenprodukt-Verbindungen der Formeln (7) und (7a) nach den Ansprüchen 6 und 13, in denen das stereogene Kohlenstoffatom, das die Gruppe R₃ trägt, die absolute Konfiguration (S) aufweist.

18. Zwischenprodukt-Verbindung der Formeln (8) und (8a) gemäß den Ansprüchen 7 und 14, in denen das stereogene Kohlenstoffatom, das die Gruppe R₃ trägt, die absolute Konfiguration (S) aufweist.

19. Verfahren nach einem der Ansprüche 8 bis 11, **dadurch gekennzeichnet, dass** man die folgenden Verbindungen herstellt:
N-{3-[2-Methoxyphenyl)sulfanyl]-2-methylpropyl}-3,4-dihydro-2H-1,5-benzoxathiepin-3-amin;
2-{[3-(3,4-Dihydro-2H-1,5-benzoxathiepin-3-ylamino)-2-methylpropyl]-sulfanyl}-6-methylphenol,
(3R)-N-{(2S)-3-[(2-Methoxyphenyl)sulfanyl]-2-methylpropyl}-3,4-dihydro-2H-1,5-benzoxathiepin-3-amin;
2-({(2S)-3-[(3R)-3,4-Dihydro-2H-1,5-benzoxathiepin-3-ylamino]-2-methylpropyl}sulfanyl)-6-methylphenol,
deren Additionssalze und die Hydrate dieser Additionssalze mit pharmazeutisch annehmbaren Mineralsäuren oder organischen Säuren.
